# EUROPEAN PATENT APPLICATION

(11) **EP 2 261 221 A1**
(43) Date of publication of application: **15.12.2010**
(21) Application number: 09714253.3
(22) Date of filing: 02.03.2009
(51) Int. Cl.: C07D 313/00, C07D 315/00, C07D 417/06, C07D 417/14, C07D 413/06, C07C 53/00, A61K 31/422, A61K 31/427, A61P 35/00

(54) **EPOTHILONE ANALOGUES, THEIR PHARMACEUTICAL COMPOSITIONS, THEIR USE AND THEIR PREPARATIONS**

(30) Priority: 29.02.2008 CN 200810082360
(71) Applicant: Tang, Li, Beijing 100176 (CN)
(72) Inventor: TANG, LI, Beijing 100176 (CN); QIU, Rongguo, Beijing 100176 (CN)
(74) Representative: Crowhurst, Charlotte Waveney
(86) International application number: PCT/CN2009/000218
(87) International publication number: WO 2009/105969

(57) **Abstract**

The present invention discloses novel Epothilone analogues, their pharmaceutical compositions, their use and their preparations, especially discloses the compounds of general formula I, their preparations and their use for preparing therapeutical compositions used as cell inhibitors.

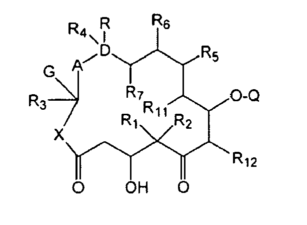

## Description

### Field of the invention

The present invention relates to novel 15-membered polyketone compounds and intermediates thereof, said polyketone compounds may refer to the derivatives of natural Epothilone in terms of their structure. The present invention also relates to their preparations and their pharmaceutical uses, particularly their uses in the preparation of a pharmaceutical composition comprising the same.

### Background of the invention

Epothilone A (EpoA) and Epothilone B (EpoB) is a 16-membered ring epoxythalidone compounds derived from macrocyclic lactone-based polyketone compounds, which, at the beginning, had been separated from soil bacteria, Sorangium cellulosum strain So ce90, having the structure below [Hofle et. al., 1996, Angew. Chem. Int. Ed. Engl. 35(13/14): 1567-1569; Gerth et. al., 1996. J. Antibiotics 49(6): 560-563].

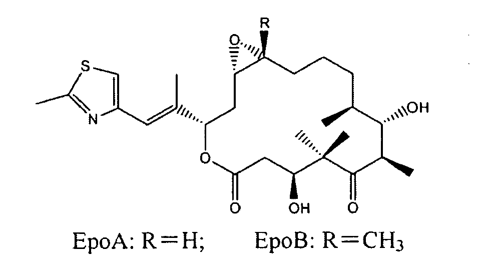

Epothilone has a great potential in the treatment of tumors. Although having different structures, the action mechanism of Epothilone family is quite similar to the well-known anti-tumor agent of paclitaxel (Taxol), including inducement of tubulin assembly and stabilization of the formation of microtubes. Those compounds exhibit powerful killing-capability on different tumor cell lines. Specifically, they exhibit remarkable effects on several multidrug-resistant tumor cell lines, especially paclitaxel-resistant tumor cell lines or tumor cell lines having resistance to other anti-tumor drugs [Altmann et. al., 2000. Biochem. Biophys. Acta. 1470(3): M79-91; Bollag et. al., Cancer Res. 55(11): 2325-2333].

The deoxygenized counterparts of Epothilones A and B, namly Epothilone C and D, have been successfully synthesized via chemical total synthesis, which, however, can also be detected along with other trace components having Epothilone-like structures in the ferment extracts of producing strain, *S*. *cellulosum*, of natural Epothilones. A t present, attentions have been focused on the development of more effective Epothilone chemotherapeutant and analogues having relative structures. For example, the naturally-occurring Epothilone compounds may be modified by chemical semi-synthesis, such as a reaction converting Epothilone B into the corresponding lactam analogue BMS247550, as described in WO99/27890.

However, it has been found that the 15-membered thalidone lactone or lactam compounds derived from Epothilone have beneficial pharmacologic properties, and can be used in the treatment of proliferative diseases. The re has been a long-lasting interest on novel Epothilone derivatives.

### Disclosure of the invention

The objective of the present invention is to provide a series of 15-membered macrocyclic thalidone lactone or lactam derivatives; and to provide a method for preparing the same, by which such novel compounds of the present invention and their novel derivatives can be obtained from compounds such as Epothilone D or B and 4-demethylated Epothilone D or B via chemical synthesis or chemical modifications and bioconversion and the like. The present invention further provides the use of such novel macrocyclic thalidone lactone or lactam compounds for the preparation of pharmaceutical compositions used for anti-tumor, inhibiting excessive growth of cells and suspending cell growth.

Polyketone compound according to the present invention, i.e. a novel 15-membered thalidone compound is represented by the following general formula I 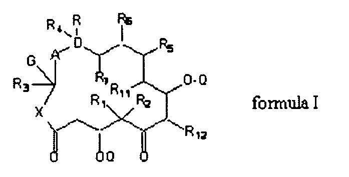 wherein,

As A-D represents a C=C bond of formula (a) or an epoxy group of formula (b) below, R₄ is not exist, 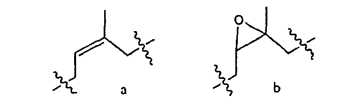
As A-D represents a C-C bond, R₄ represents a hydroxy group or H,
G is selected from a substituted or unsubstituted alkyl group, a substituted or unsubstituted aryl group, a heteroaryl group, a heterocyclic group, a cycloalkyl group, or any one selected from the following formulae: 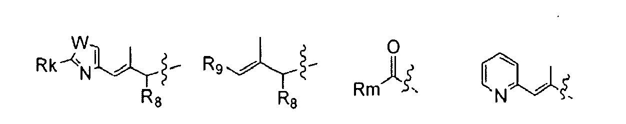 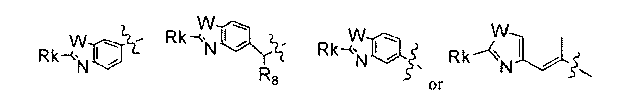
Q is selected from H, a C₁₋₄ alkyl group, NH₂ or a hydroxy-protecting group such as silyl ether selected from any one of TMS, TES or TBS:
R₁, R₂ are each independently selected from H or a substituted or unsubstituted alky group (preferably a C₁₋₄ alkyl group, more preferably methyl group), or together form a cycloalkyl group;
R₈ is selected from H, a hydroxy group, a substituted or unsubstituted alky group (preferably a C₁₋₄ alkyl group, more preferably methyl group) or NH₂, N₃ or NR₁₃R₁₄;
X represents O, S or N-R₁₅, wherein R₁₅ represents H, NR₁₆R₁₇, a substituted or unsubstituted alky group (preferably a C₁₋₄ alkyl group, more preferably methyl group), a substituted or unsubstituted aryl group, a cycloalkyl group or a heterocyclic group;
Rm is selected from H, methyl, NR₁₆R₁₇ or halomethyl;
R₁₂ is selected from H, an allyl group, a hydroxy group, NH₂ or a substituted or unsubstituted alky group (preferably a C₁₋₄ alkyl group, more preferably methyl group); preferably, R₁₂ is an allyl group;
R₉ is selected from H, a substituted or unsubstituted alky group (preferably a C₁₋₄ alkyl group, more preferably methyl group), aryl group, a heteroaryl group, a cycloalkyl group or a heterocyclic group, said heteroaryl group is prefereably selected from thiazolyl, pyridyl, oxazolyl, isoxazolyl, quinoline or benzoxazolyl;
R₃, R₄, R₅, R₆, R₇, R₁₁, R₁₃, R₁₄, R₁₆, R₁₇ are each independently selected from H, a hydroxy group, NH₂ or a substituted or unsubstituted alky group (preferably a C₁₋₄ alkyl group, more preferably methyl group), wherein R₅, R₆ may also together form a C=C bond;
Rk is selected from H, methyl, a substituted or unsubstituted alky group (preferably a C₁₋₄ alkyl group, more preferably methyl group), an aminoalkyl group, a hydroxyalkyl group or a haloalkyl;
R is selected from H, trifluoromethyl, a substituted or unsubstituted alky group (preferably a C₁₋₄ alkyl group, more preferably methyl group) or halogen;
W represents S or O, NH, N-alkyl;
or pharmaceutical acceptable salts, hydrates, polycrystalline structures, optical isomers, racemates, diastereomers or enantiomers of said compound.

Preferably, G in compound of formula I is selected from:

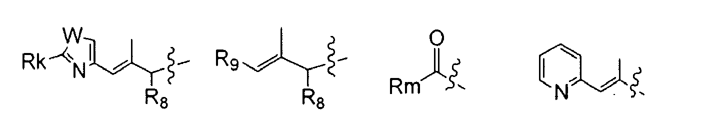

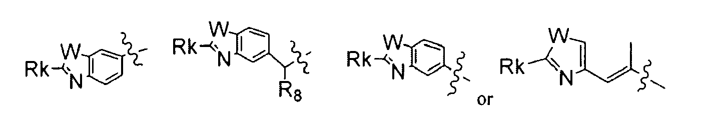

In one embodiment, compound of the present invention is represented by the structure of the following general formula II: 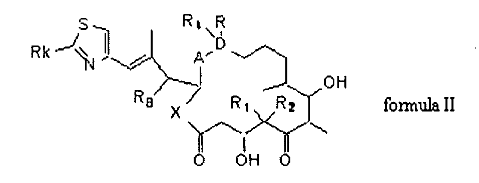
wherein, X is NR₁₅ or O;
R₈ is NHR₁₅ or OQ; each of the other groups has the same meaning as defined above.

In another embodiment, compound of the present invention is represented by the structure of the following general formula III: 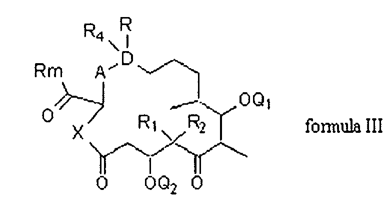 wherein, Q₁ and Q₂ each represents H, a C₁₋₄ alkyl group, NH₂ or hydroxy-protecting group such as silyl ether selected from any one of TMS, TES or TBS; each of the other groups has the same meaning as defined above.

In another embodiment, compound of the present invention is represented by the structure of the following general formula IV: 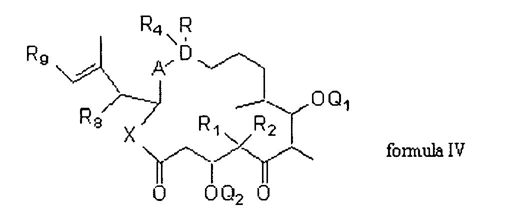 wherein, each group in formula IV has the same meaning as defined above.

In yet another embodiment, compound of the present invention is represented by the structure of the following general formula V: 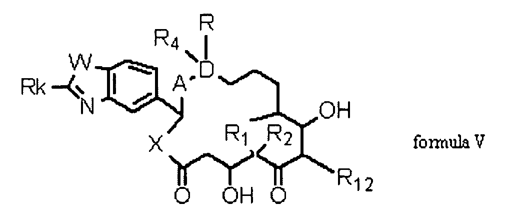 wherein, X is NR₁₅ or O; R₁₅ is H, a methoxy group or an alkyl group; R₁₂ is H, an allyl group, a substituted or unsubstituted alkyl group (preferably a C₁₋₆ alkyl group, more preferably methyl group)

In still another embodiment, compound of the present invention is represented by the structure of the following general formula VI: 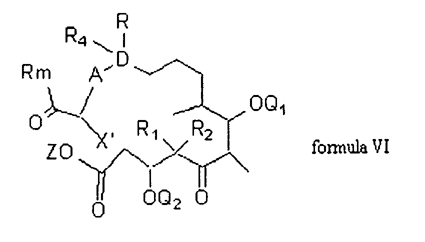 wherein
X' is NHR₁₅, NR₁₅P, OH or OQ; wherein, R₁₅ is H, a methoxy group or an alkyl group; P is a N-protecting group;
Z is H, a substituted or unsubstituted alkyl group (preferably a C₁₋₆ alkyl group, more preferably methyl, tert-butyl), a cycloalkyl group, an aryl group or a carboxyl-protecting group;
Q₁ and Q₂ each independently represents H, a C₁₋₄ alkyl group, NH₂ or a hydroxy-protecting group.

Unless otherwise indicated, the terms as used herein are of the following meanings:

Any asymmetric carbon atoms may be in the configuration of (R)-, (S)- or (R, S)-, therefore, the compound of the present invention may present in a larege amount of variety form of optical isomers, geometical isomers and stereoisomers, and that all these isomers and mixtures thereof are included in the scope of the present invention.

Unless otherwise indicated, alkoxyl, alkyl (including alkyl such as hydroxyalkyl, haloalkyl and aminoalkyl) defined in the compound of the present invention preferably refers to a lower alkyl or a lower alkoxyl, "lower" refers to a group having 6 or less carbon atoms, preferably a group having 4 or less carbon atoms, wherein said group may be a straight chain or a branched chain having one or more branches. Substituted alkyl group refers to an alkyl group substituted by 1 to 4 subsituents, said substituent may be a halogen, trifluoromethane, trifluoromethoxy, acyl, aryloxy, amino, alkylamino, heterocyclic amino, arylamino, arylalkylamino, acylamino, alkylsulfonyl, alkythio, alkoxycarboxyl etc. Aryl group refers to a monocyclic or bicyclic aryl group having 6-12 carbon atoms on the ring, such as phenyl, diphenyl etc, aryl group may be a substituted or unsubstituted aryl group. Substituted aryl group refers to an aryl group substituted by 1 to 4 subsituents, said substituent may be, for example, a substituted or unsubstituted alkyl group, a halogen, CF₃, trifluoromethoxy, hydroxy, alkoxyl, cycloalkoxyl, cycloalkylamino, acyl, aryloxy, amino, alkylamino, heterocyclic amino, arylamino, arylalkylamino, acylamino, alkylsulfonyl, mercapto, alkythio, cycloalkythio, nitro, carboxyl, carboxyalkyl, carbamyl, alkoxycarboxyl etc. Heteroaryl group refers to any 5-membered or 6-membered ring having 1 to 3 heteroatoms selected from nitrogen, oxygen and/or sulfur, wherein 5-membered ring has 0 to 2 double bonds, while 6-membered ring has 0 to 3 double bonds. H eteroatom like nitrogen and sulfur may be optionally oxidized, and also optionally quatemized Unsaturated heteroaryl group of a monocyclic or bicyclic aryl group having at least one nitrogen atom and 0 or 1 oxygen atom and 0 or 1 sulfur atom is preferable, a group having 5 to 12, more preferably 5 or 6, ring atoms on the connecting ring is more preferable, and that said heteroaryl group may be unsubstituted or substituted by one or more substituents preferably selected from halogen, alkoxyl, alkythio, hydroxy, alkyl and/or acyl. Heteroaryl group is preferably selected from thiazolyl, pyridyl, oxazolyl, quinoline or benzoxazolyl or benzothiazolyl. Cycloalkyl group refers to a saturated carbon ring that may be optionally substituted, having preferably 1 to 3 rings, and 3 to 7 carbon atoms on each ring, which may fuse together with an unsaturated C₃-C₇ carbon ring. Cycloalkyl group is preferably cyclopropyl, cyclopentyl etc. Heterocyclic group refers to a saturated non-aromatic cyclic group that may be optionally substituted, such as 4- to 7-membered monocyclic ring, 7-to 11-membered bicyclic ring, or 10- to 15-membered tricyclic ring, and that at least one heteroatom is present on at least one of the rings, and 1, 2, or 3 heteroatoms selected from nitrogen, oxygen, sulfur may be present in the ring of each heterocyclic group, wherein heteroatom like nitrogen and sulfur may be optionally oxidized, while nitrogen atom may be optionally quaternized, heterocyclic group may be linked on any heteroatomes or carbon atoms. Heteroaryl group may fuse with an unsaturated C₃-C₇ carbon ring, said fused group may be, for example, pyazolidyl, thiazolyl, pyridyl, oxazolyl, isoxazolyl, quinoline, benzoxazolyl or benzothiazolyl, imidazolyl and furanyl.

Hydroxy-protecting group, N-protecting group and carboxyl-protecting group used in the compounds of the present invention refer to the protecting group commonly employed in the relevant art, for example, hydroxy-protecting group is preferably silyl ether, such as TMS, TES or TBS; N-protecting group is preferably tert-butylcarboxylate; carboxyl-protecting group is preferably methyl, tert-butyl (e.g. tert-butyl formed from tert-butanol and carbodiimide) and the like.

D uring the process for preparing the compound of the present invention, functional groups in the starting compounds that do not involve in the reaction may be unprotected dependent on the particular process step to be taken place, or they may be protected by one or more protecting groups, or completely or partly removed. The protecting groups are **characterized in that** they get easily to be removed by solvolysis, reduction, and photolysis or by enzyme activity, and that they do not remain in the end products. H ydroxy-protecting group is preferably a lower alkylsilyl typed hydroxy-protecting group as described herein, and they can be introduced and removed in the same manner as the method described herein from necessity, selective protection or deprotection is also possible. Herein, protecting groups are not mentioned in some places to be suitably used, however, a person skilled in the art may realize the suitable time or way for use of the protecting group.

Molecular cyclization may be performed under conventional conditions. F or example, if X' is a hydroxy group, cyclization refers to macrocyclic lactonization, for example, if X' is NH₂ or NHR (alkyl), cyclization refers to macrocyclic lactamization. Macr ocyclic lactonization takes place in a suitable solvent or solvent mixture from precusor of acid (or anhydride) compounds (or from protected derivatives) with free hydroxy group under the condition as described in M. Yamaguchi et. al., Bull. Chem. Soc. Jpn. 1979, 52:1989. Lactamization (macrocyclic lactamization) is performed under the conventional condition for linking carboxylic acid and amide, particularly using a standard coupling agent such as DCC/HOBt or diphenylphosphoryl azide or bromo-tris-pyrrolidino-phosphonium hexafluorophosphate (PyBroP) commonly employed in peptide chemisty for the conversion of a corresponding macrocyclic lactam.

Compound of formula I, where A-D is an epoxy and C-C bond, can be prepared from compound of formula I with A-D being a C=C bond by known method for epoxidation in the relevant art. If peroxide is used, reaction with dioxirane is preferably taken place in a suitable solvent or solvent mixture at a relatively lower temperature.

A preferable compound involves in some embodiments of the present invention, when G is of the following, 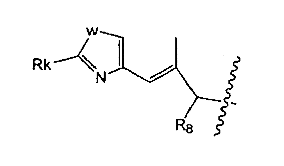 a compound represented by the structure of the following formula II and salts thereof are provided: 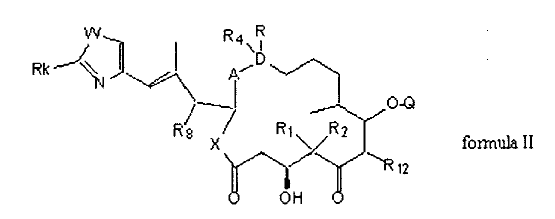 wherein, compound of general formula II can be prepared from Epothilone or derivatives thereof LL by chemical modification or/and bioconversion, such as synthetic route of reaction 1:

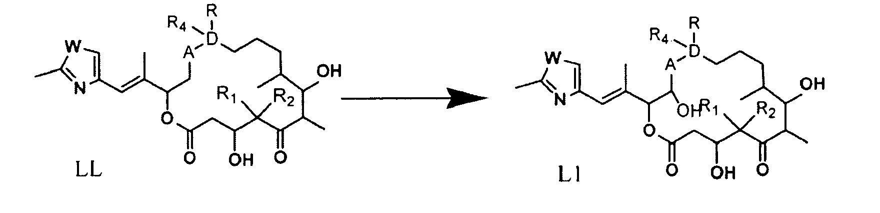

C -14 of Epothilone or derivatives thereof is hydroxylated by using hydroxylated enzyme derived from microorganisms, for example, by synthetic route of reaction 1. Such a C-14 hydroxylated Epothilone derivative L1 can be prepared by microbialconversion as described in Example 1. According to the description disclosed in CN1629283 (published on June 22, 2005), Epothilone C or D can be easily obtained. For example, Epothilone C or D is produced as the main metabolite from the producing strain of natural Epothilone A and B caused by the inactivation of the P450 gene of Epothilone biosynthetic gene. According to the description disclosed in CN1521258 (published on August18, 2004), 4-demethylated Epothilone A and B or C and D can be easily obtained. Under general circumstances, inactivation of MT domain (methyl-transferring domain) in extender module 8 of Epothilone biosynthetic gene results in the production of 4-demethylated Epothilone as the main metabolite from the producing strain of natural Epothilone. These two patent documents are incorporated herein by reference. O n the other hand, the present invention relates to a compound of structural formula II, wherein W is O, synthesized from the oxazole counterpart of Epothilone derivative. According to the description of CN1521258, by introducing an excess amount of serine to the protecting strain of Epothilone, the producing strain normally for the production of the thiazole Epothilone compound can be adjusted in a manner that facilitates the production of the oxazole counterpart.

Compound L4, a preferable compound of general formula II can be prepared from C-14 hydroxylated Epothilone derivatives according to the general procedure described in synthetic route of reaction 2. Such compound L4 can be prepared according to the procedure described in Example 2.

### Synthetic route of reaction 2:

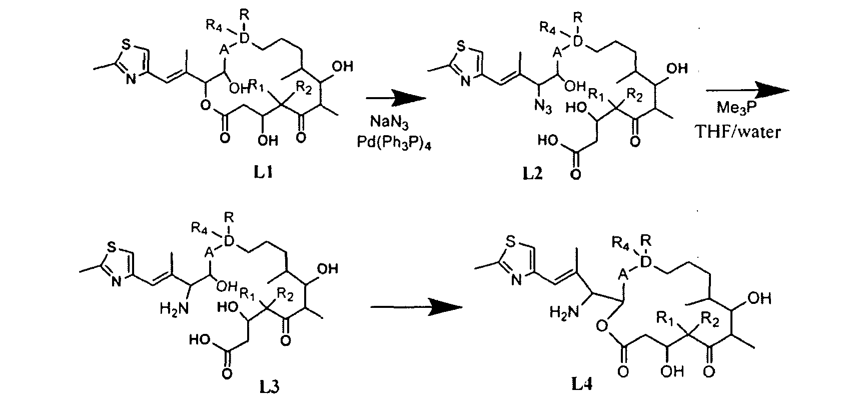

1. Allyl palladium π counterpart is formed by reacting 14-OH Epothilone and its derivatives with tetrakis(triphenyphosphine)palladium in THF/water, which is then treated with sodium azide, and results in the formation of a ring-opened azide compound.
2. Next, azide is reduced to NH₂ by using Adam's catalyst (PtO₂) or reducing agent such as triphenylphosphine in ethanol.
3. Carboxylic acid and 14-OH in L3 are subjected to macrocyclic lactonization. Under Yamaguchi condition, hydroxy acid is treated with macrocyclic lactonization agent, such as 1,3,5-trichlorobenzoyl chloride, and triethylamine in OC and THF, followed by addition of a solution of 4-(dimethylamino)pyridine in toluene to the reacting mixture, and the temperature is elevated to 75°C, and compound L4 or intermediate (protected L4) is obtained.
4. Protecting group may be present in the 14-OH Epothilone precursor, and to protect those relevant functional groups so as to prevent any undersirable side reactions, such as acylation, etherification, oxidation, solvolysis and the like. Protecting group is **characterized in that** they get easily to be removed by solvolysis, reduction, and photolysis or by enzyme activity, and that they do not remain in the end products. For example, firstly, protecting group can be present in the 3-, 7-, and 14-OH free hydroxy group of the 14-OH Epothilone precusor; they are P1, P2 and P3 respectively. Before macrocyclic lactonization, protecting group P3 of 14-OH can be selectively removed by AcOH in THF under the condition that protecting groups P1, P2 are not removed. Finally, carboxylic acid can react with 14-OH undergoing macrocyclic lactonication to obtain the intermediate, protected L4. P1 and P2 can be removed by any means commonly employed in the relevant art. In the case P1 and P2 is silyl ether, such as TMS, TES or TBS, deprotection can be performed by treating with acid, such as HF in dichloromethane, pyridine or trifluoroacetic acid and to afford L4.

### Synthetic route of reaction 2-B:

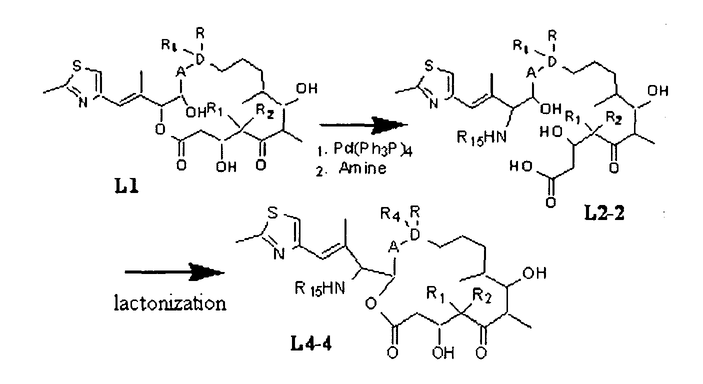

In the case R8 in general formula II is NHR₁₅ and R₁₅ is not H, compound of general formula II can be prepared from C-14 hydroxylated Epothilone derivatives according to the general procedure described in synthetic route of reaction 2-B.
1. Firstly, allyl palladium π counterpart is formed by using tetrakis(triphenyphosphine)palladium, and then treated with primary amine to afford L4-, i.e. with R15NH-, wherein R15 is OH, a substituted or unsubstituted alkyl group, a cycloalkyl group, an aryl group, a heteroaryl group, O-alkyl.
2. According to the condition described in M. Yamaguchi et. al., Bull. Chem. Soc. Jpn. 1979, 52:1989, macrocyclic lactonization is peformed.
3. If R15 is OH, protecting group has to be removed from R15-O-TMS to afford the end product.

Compound L7, a preferable compound of general formula II can be prepared from C-14 hydroxylated Epothilone compound according to the general procedure described in synthetic route of reaction 3, as described in Example 3.

### Synthetic route of reaction 3:

1. Firstly, free hydroxy group, 3- and 7-OH, can be protected by a suitable group, 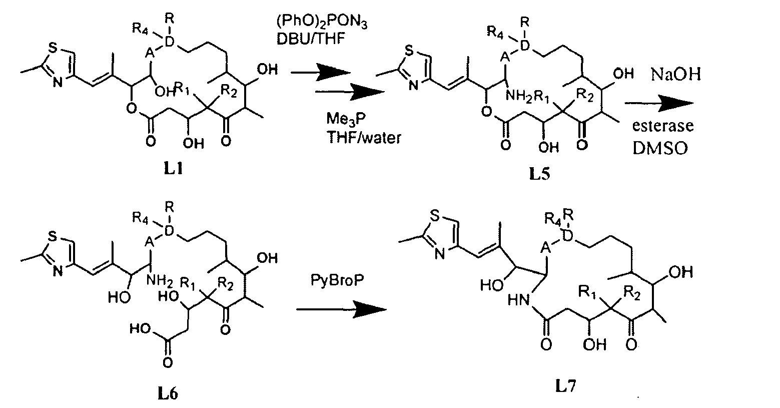 such as triethylsilyl, butyldimethylsilyl etc., respectively P1 and P2. Protecting group P3 of 14-OH can be selectively removed by using AcOH in THF under the condition that protecting groups P1, P2 are not removed.
2. Treating the 3,7-protected Epothilone derivative with tetrakis(triphenyphosphine)palladium to form the allyl palladium π counterpart, followed by treating with primary amine, or reacting with diphenylphosphoryl azide and diazabicyclo(5.4.0)undec-7-ene (DBU) to afford 14-azide Epothilone. Next, reduction is performed by using trimethylphosphine and NH4OH aqueous solution to afford 3,7-protected 14-amino-Epothilone derivative.
3. Under the condition that allows the selective reduction of lactone carboxyl, 3, 7-protected 14-amino-Epothilone derivative is reacted with a reducing agent such as di(isobutyl)aluminium hydride (DiBAI-H) to afford the protected ring-opened intermediate.
4. Corresponding macrocyclic lactonization of the protected ring-opened intermediate is performed using a standard amide coupling agent such as diphenylphosphoryl azide and sodium hydrogen carbonate or EDC/HOBT (1-hydroxybenzotriazole) and 1-(3-dimethylaminopropyl)-3-ethylcarbodiimide in DMF or bromo-tris-pyrrolidino-phosphonium hexafluorophosphate (PyBroP) to afford the intermediate, protected L7. Deprotection is performed to afford L7compound.

Compound L9, a preferable compound of general formula II can be prepared from C-14 hydroxylated Epothilone compound according to the general procedure described in synthetic route of reaction 4.

### Synthetic route of reaction 4:

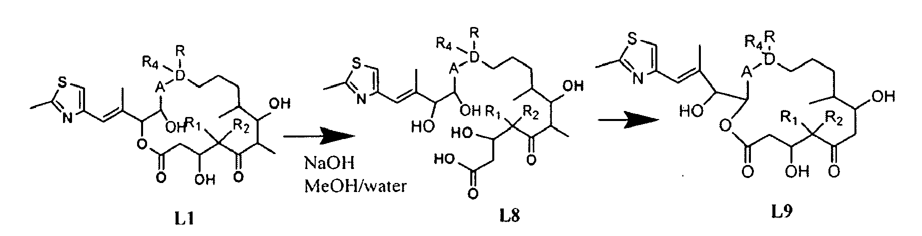

Make reference to synthetic route of reaction 4, the corresponding ring-opened Epothilone hydroxy acid intermediate prepared by saponification is used to prepare compound L9 of the present invention. For example, Epothilone derivatives are converted to a ring-opened seco-acid hydroxy acid by hydrolysis by treating with a solution of sodium hydroxide in methanol/water or esterase (such as Pig liver esterase) in DMSO. Finally, 14-OH and carboxylic acid are subjected to lactonization according to the method disclosed by Yamaguchi *et. al.* to afford L9. Technically, the ring-opened Epothilone hydroxy acid intermediate may be converted to tert-butyl ester using tert-butanol, carbodiimide (such as dicyclohexyl carbodiimide) and 4-(dimethylamino)pyridine as catalyst, or said hydroxy acid may be converted to methyl ester by reacting with trimethylsilyl diazomethane. For example, 15-OH of the methyl ester can be protected by silylation, for example, by treating with trimethylsilyl chloride/trimethylsilyl imidazole. The protecting group of butyl ester or methyl ester in the intermediate can be removed by treating with camphorsulfonic acid (CSA) in methanol and dichloromethane or with sodium hydroxide, followed by treating with acetic acid. After subjecting 14-OH and carboxylic acid to macrocyclic lactonization, intermediate, i.e. L9 having the free hydroxy group protected is obtained, and then L9 is obtained after deprotection.

A preferable compound involves in some embodiments of the present invention, when G is of the following, 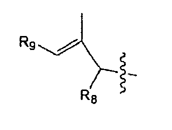 a compound represented by the structure of the following formula III and salts thereof are provided:

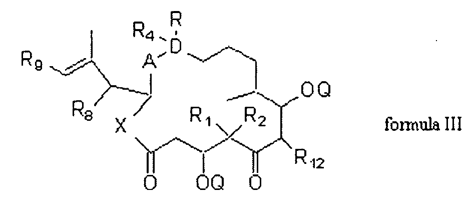

Compound of general formula III can also be prepared from Epothilone D or derivatives thereof such as 4-demethylated Epothilone D according to the general procedure described in synthetic route of reaction 5, as described in Eample 4.

### Synthetic route of reaction 5:

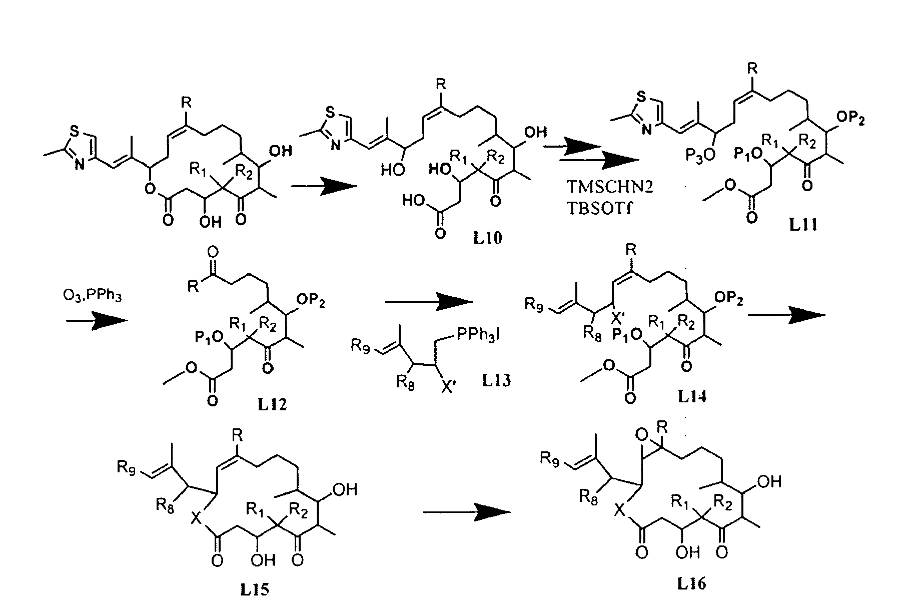

wherein, X' is OP3, NR15P or SP', P1 and P2 each independently represents H or the same or different protecting group, P3 is H or a protecting group, P is H or N-protecting group, P' is H or S-protecting group. X is O, NH, NR₁₅ or S.
1. The corresponding ring-opened Epothilone hydroxy acid intermediate prepared by saponification is used. For example, Epothilone derivative is converted to a ring-opened seco-acid hydroxy acid by subjecting it to hydrolysis by treating with an aqueous solution of sodium hydroxide in methanol or with a suitable esterase (e.g. Pig liver esterase etc).
2. The ring-opened Epothilone hydroxy acid intermediate can be converted to methyl ester by reacting with an alkylating agent such as trimethylsilyl diazomethane (TMSCHN₂). For example, 3-, 7- and 15-free hydroxy group of methyl ester can be protected (PI, P2 or/and P3) by silylation by treating with trimethylsilyl chloride/trimethylsilyl imidazole introducing the same or different protecting group such as t-butyldimethylsilyl trifluoromethane sulfonate.
3. Double bond at C12 in protected L11 is subjected to oxidative cleavage by using ozone to afford compound L 12.
4. L12 and compound L13 are subjected to Wittig olefination to afford L14.
5. Protecting group of methyl ester can be removed by treating with a particular type of base such as hydroxide (LiOH), and to afford a carboxylated ring-opened Epothilone derivative. The selectively deprotected 14-X' and 1-carboxylic acid are subjected to macrocyclic lactonization or lactamization, to afford L15 with 3-, 7-protected or deprotected.
6. Finally, vinyl group of L15 is subjected to epoxidation to afford L16.

A preferable compound involves in some embodiments of the present invention, when G is of the following 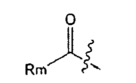 a compound represented by the structure of the following formula III and salts thereof are provided:

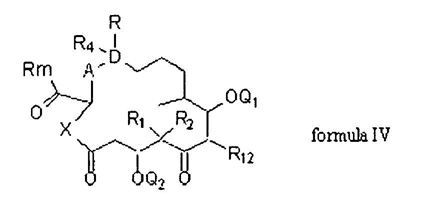

### Synthetic route of reaction 6:

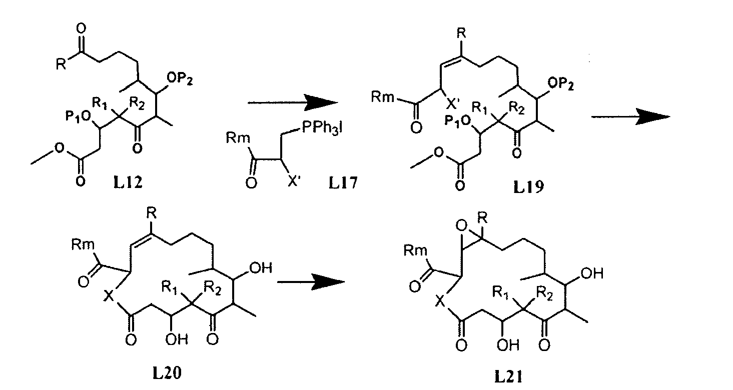

Compound of general formula IV can also be prepared from L12 and L17 (preferably Rm=CH3) according to the general procedure described in synthetic route of reaction 6. The synthetic procedure is equivalent to the general procedure 4, 5, 6 described in synthetic route of reaction 5.

### Synthetic route of reaction 7:

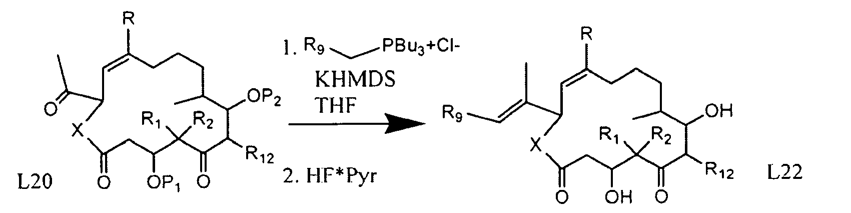

In other embodiments of the present invention, compound L22 can be prepared from compound of formula IV according to the synthetic proedure described in synthetic route of reaction 7. See example 5, wherein R9 is prefereably

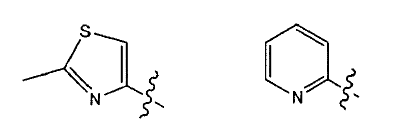

For example, as described in A.Rivkin et. al., J.Am.Chem.Soc. 2003, 125:2899, ketone compound L20, where P1 and P2 is a hydroxy-protecting group, reacts with a suitable Wittig inner salt to obtain a protected compound, and compound L22 is obtained after deprotection.

Wittig inner salt can be prepared by reacting a corresponding phosphonium with a strong base such as potassium bis(trimethylsilyl)amide (KHMDS) or sodium bis(trimethylsilyl)amide (NaHMDS), butyl lithium, sodium hydride or the like. Alternatively, Wittig inner salt can be prepared by other methods known in the art. Phosphonium can be prepared by reacting alkyl halide with triarylphosphine or trialkylphosphine (e.g. triphenylphosphine or tributyl phosphine), see Example 6.

In some embodiments of the present invention, phosphonium L23 (when L13 is L23, R8 in L13 is OP3) can be prepared according to synthetic route of reaction 8.

### Synthetic route of reaction 8:

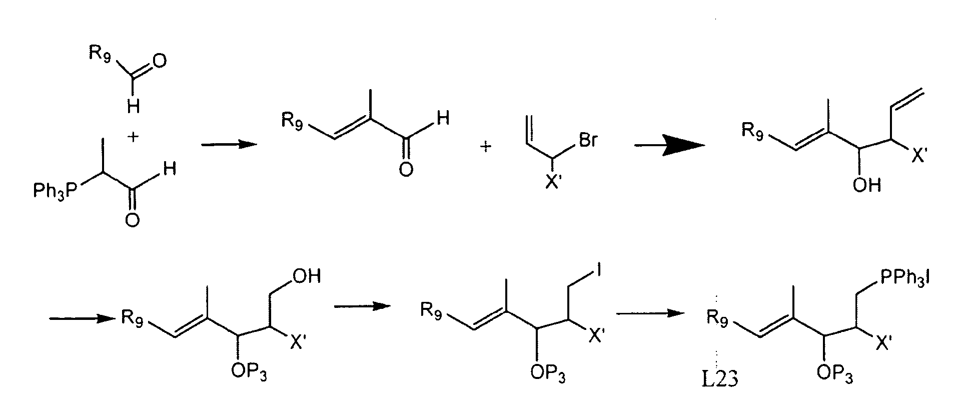

According to standard Wittig olefination (Meng, D., et. al. J. Org. Chem., 1996, 61: 7999)
1. Treating with organometallic reagent such as X'-allyl magnesium bromide.
2. Protecting the free hydroxy group by TES protecting group (OP3) using triethylsilyl chloride in DMF. Reacting with AD-mix-a by Sharpless, followed by oxidation of the cleaved bond using lead tetraacetate in ethyl acetate, and subjected to reduction using reducing agent such as sodium borohydride in methanol.
3. Reacting I, imidazole and triphenylphosphine in toluene.
4. Reacting triphenylphosphine in acetonitrile under reflux.

In some embodiments of the present invention, phosphonium L24 (when L13 is L24, R8 in L13 is NR15P) can be prepared according to synthetic route of reaction 9.

### Synthetic route of reaction 9:

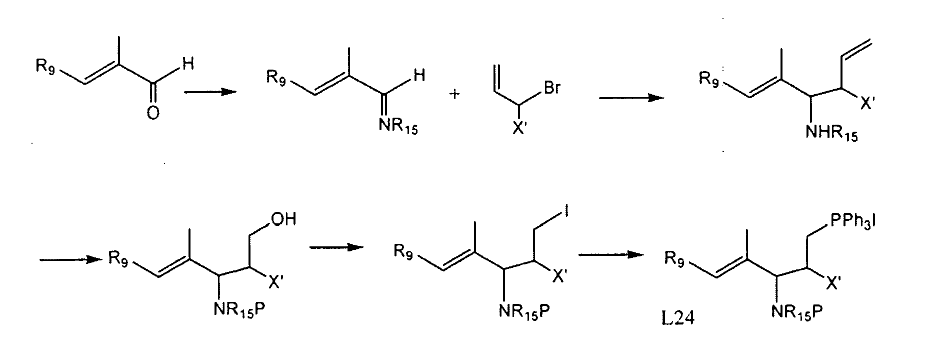

1. Prepared by treating with amine under anhydrous condition, e.g. using a catalytic amount of p-toluenesulfonic acid and subjected to azeotropy to remove water.
2. Treating with allylating agent such as 3-X'-allyl magnesium bromide.
3. The rest of the synthetic procedure is equivalent to the general procedure 3, 4, 5 described in synthetic route of reaction 8.

In some embodiments of the present invention, in the case R9 in L13 is thiazole or pyridine or the starting materials of synthetic route of reaction 9 are thiazole or pyridyl aldehyde compounds, thiazolyl aldehyde or pyridyl aldehyde can be prepared according to known method (Taylar, R.E. Tetrahedron Lett. 1997, 38:2061) or according to synthetic route of reaction 9-B, and then phosphonium compound, where R9 is thiazole or pyridine, can be prepared according to synthetic route of reaction 8 or 9.

### Synthetic route of reaction 9-B:

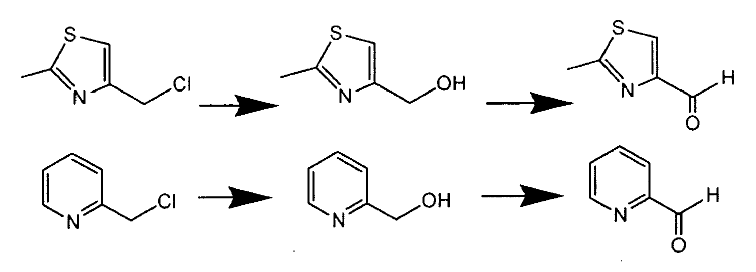

In some embodiments of the present invention, phosphonium L25 (when L17 is L25, X'in L17 is NR15P, R8 is methyl) can be prepared according to synthetic route of reaction 10.

### Synthetic route of reaction 10:

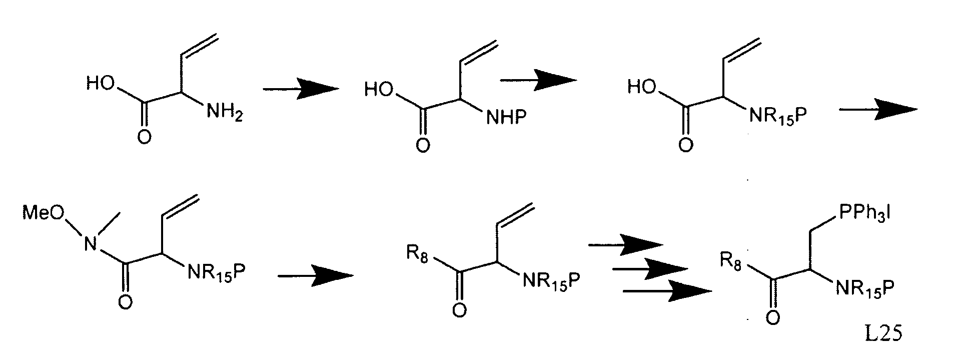

1. Using vinyl glycine for N-protecting, P is t-butyloxycarbonyl suitable for N-protecting.
2. When R15 is not H, compound is N-alkylated using haloalkane in the presence of base such as sodium hydroxide.
3. Treating with N.O-dimethylhydroxyamine and standard coupling agent such as EDCI and HOBT.
4. Treating with organometallic reagents such as alkyl or aryl magnesium halide or hydroxamic acid.
5. The rest of the synthetic procedure is equivalent to the general procedure 3, 4, 5 described in synthetic route of reaction 8.

In some embodiments of the present invention, phosphonium L26 (when L17 is L26, X'in L 17 is OP3, R8 is methyl) can be prepared according to synthetic route of reaction 11.

### Synthetic route of reaction 11:

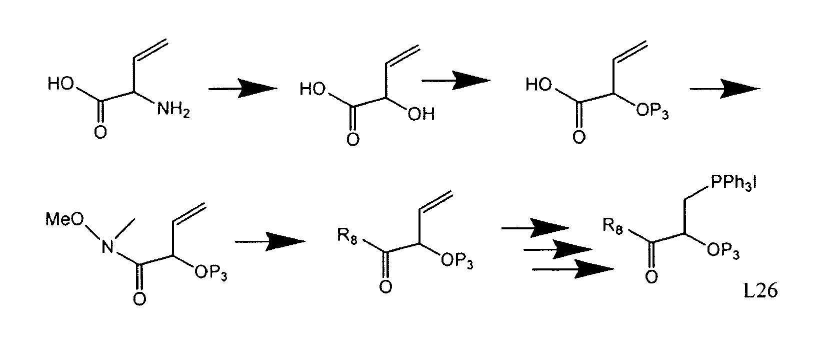

1. Treaing vinyl glycine with nitric acid.
2. Protecting the free hydroxy group by TES protecting group (OP₃) using triethylsilyl chloride in DMF.
3. Treating with N.O-dimethylhydroxyamine and standard coupling agent such as EDCI and HOBT.
4. Treating with organometallic reagents such as alkyl or aryl magnesium halide or hydroxamic acid.
5. The rest of the synthetic procedure is equivalent to the general procedure 3, 4, 5 described in synthetic route of reaction 8.

Preferable compound of general formula I of the present invention and salts thereof can also be prepard by the total sythesis as shown in synthetic route of reaction 12 and 12B.

### Synthetic route of reaction 12:

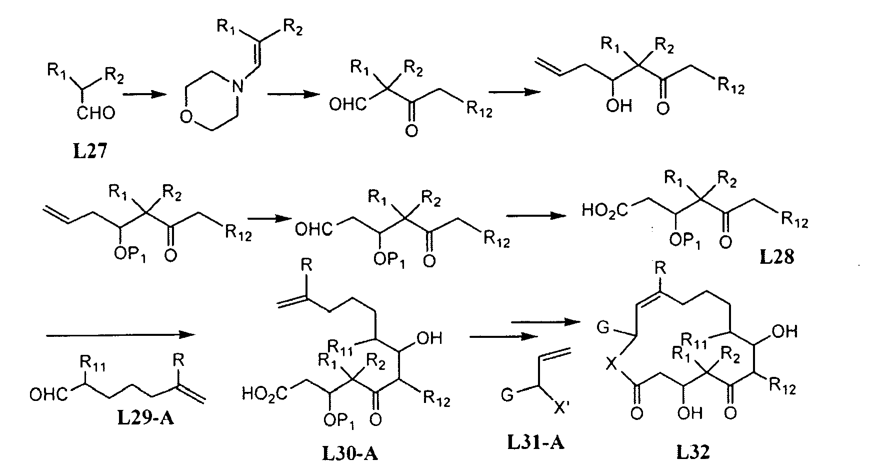

### Synthetic route of reaction 12B

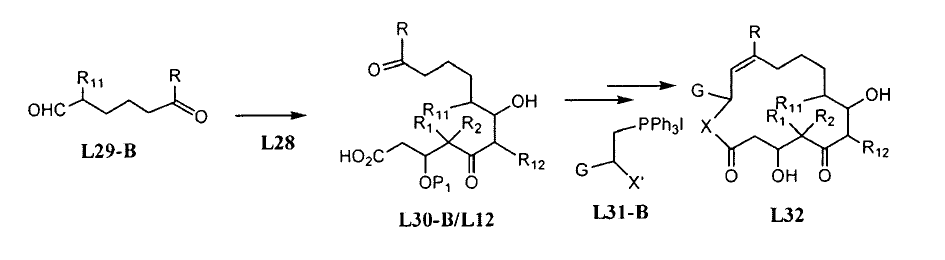

For example, compound L28, where P1 is an O-protecting group such as tertbutyldimethylsilyl, can be prepared from compound L27 according to known method (i.e. Nicolaou, K.C. et. at., Angew.Chem. Int. Ed. Engl. 1997, 36:166). Compound 29 can be prepared by know method (i.e., Schinzer, D. et. at., Eur.Chem.Chron. 1996, 1:7). Aldol reaction of compounds L28 and 29-A can afford compound L30-A. W hen X'in L31 is -OH, compound L30 and compound of 31 is coupled by using standard esterifying agent such as DCC and DMAP; or when X' in L31 is NHR15, compound L30-A and compound L31-A is coupled by using standard amide coupling agent such as DCC, BOP, EDC/HOBT, PyBroP; compound L32 can be prepared by olefin ring-closing metathesis using Grubbs' catalyst (RuCl₂(=CHPh)(PCY₃)₂; see Grubbs. et. al.. Angew. Chem. Int. Ed. Engl. 1995, 34:2039 or Schrock's catalyst (see Schrock,R.R. et.at., J. Am. Chem. Soc. 1990, 112-3875) (Synthetic route of reaction 12).

Alternatively, compound L30-B (when R11, R12 is methyl, L30-B is L12) can be prepared by Aldol reaction of compound L28 and compound of 29-B. Compound L30-B and compound L31-B can be coupled appropriately by Wittig olefination, followed by macrocyclic lactonization or lactamization. When X' in L31 is -OH, compound L32 can be subjected to macrocyclic lactonization by reacting with a standard esterifying agent such as DCC and DMAP; or when X' in L31 is NHR15, compound L32 can be obtained by macrocyclic lactamization using standard amide coupling agent such as DCC, BOP, EDC/HOBT, PyBroP. See synthetic route of reaction 12B.

### Synthetic route of reaction 13:

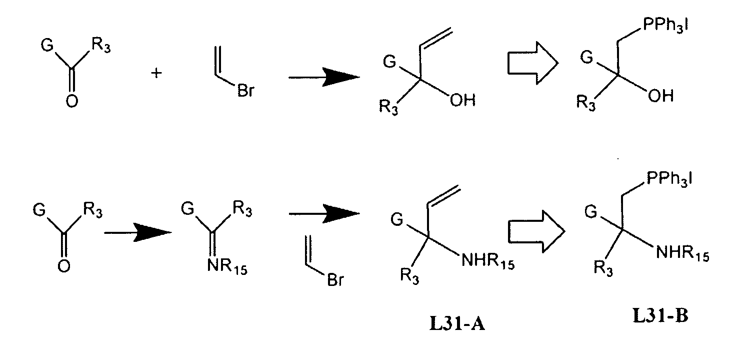

Compound L31 can be prepared from aldehyde compound following the synthetic reaction as shown in synthetic route of reaction 13. Aldehyde compound, where G is a substituted or unsubstituted alkyl, aryl, heteroaryl, dicyclic heteroaryl group or G is preferably as shown below, is vinylated by treating with a vinylating agent such as vinylmagnesium bromide, to afford compound L31-A where X' is OH; when X' in L31-A is NHR15, compound G is vinylated by reacting aldehyde compound, where G is a substituted or unsubstituted alkyl, aryl group, heteroaryl group, dicyclic heteroaryl group or G is preferably as shown below, with amine under anhydrous condition, followed by treating with a vinylating agent such as vinylmagnesium bromide, to afford compound L31-A where X' is NHR15. Phosphonium L31-B can be prepared from compound L31-A according to the procedures equivalent to the general procedure 3, 4, 5 described in synthetic route of reaction 8.

G is preferably of the following formula:

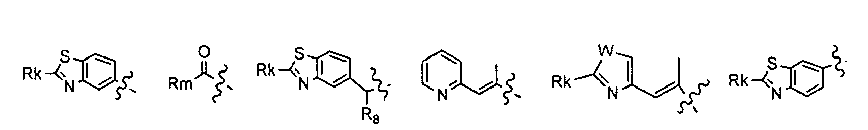

When G is a benzothiazole, benzothiazole aldehyde compound can be prepared according to the synthetic route of reaction 14 below, or aldehyde compound L33 can be prepared as described in example 8, compound L31 can be prepared following the synthetic route of reaction 13.

### Synthetic route of reaction 14:

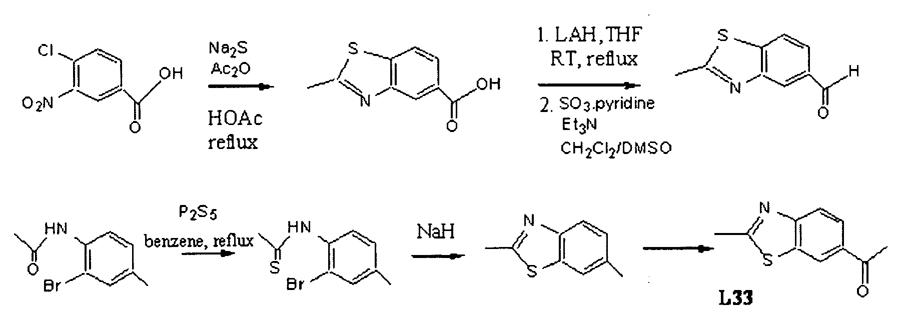

Compound of general formula I is preferably an epoxide (12, 13-epoxy derivatives). Such compound can be prepared according to the epoxidation as shown in equation 15 of the present invention described in Example 9.

### Equation 15:

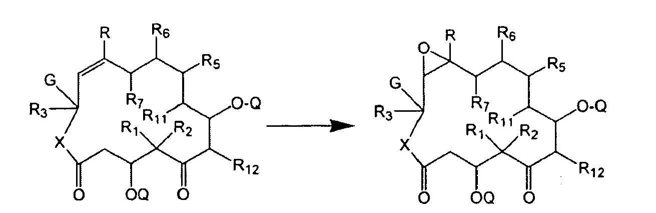

Compound of general formula 1 is preferably a 12-hydroxylated derivative. C-12 hydroxy compound of formula I can be prepared according to the general procedure for chemical modification described in equation 16.

### Equation 16:

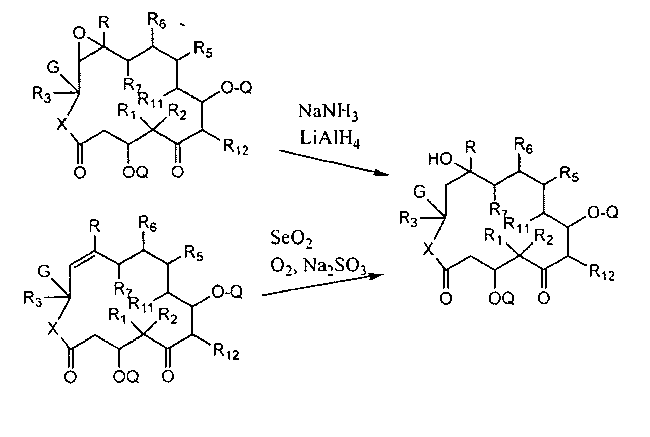

In other embodiments, the present invention provides a compound of general formula I having the following structures.

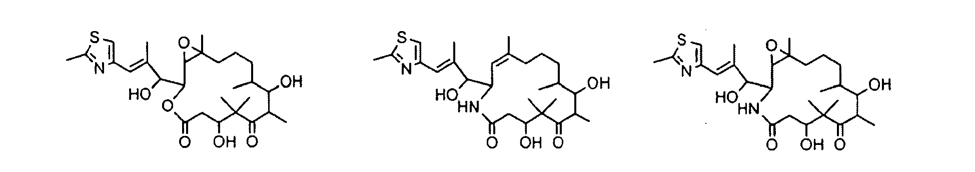

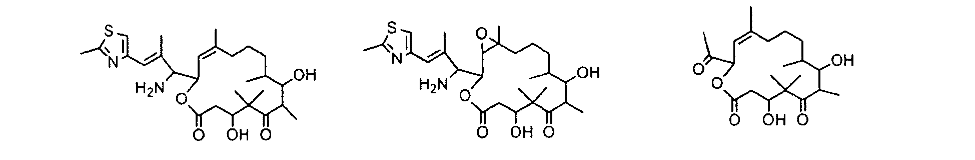

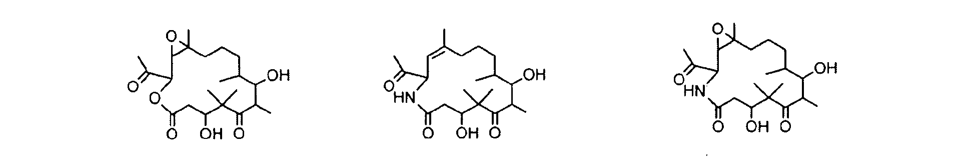

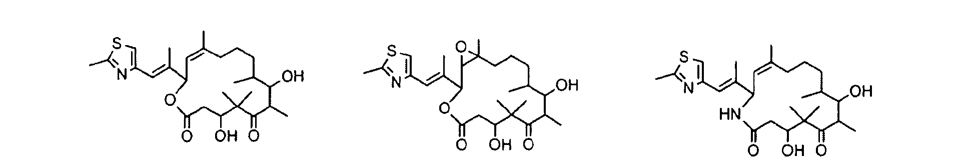

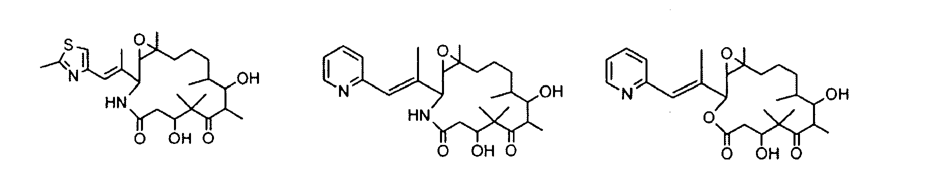

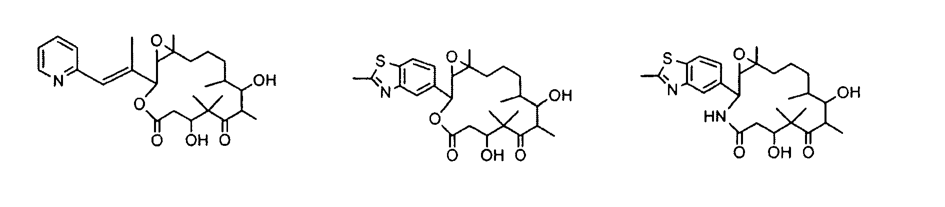

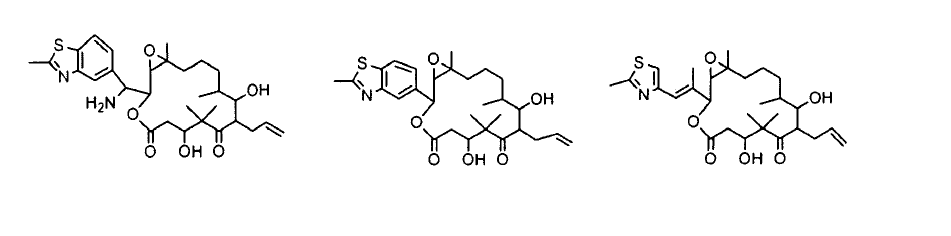

The compounds of the present invention can be screened by conventional analytical methods known in the art. For example, cytotoxicity of compound can be determine according to SRB analysis described in Skehan et. al., J. Natl. Cancer Inst. 1990, 82: 1107, which is incorporated herein by reference.

The person skilled in the art is able to screen the compound of the present invention for tubulin polymerization using the conventional analytical method known in the art. For example, screening compound for tubulin polymerization according to the method described in Gianakakou et. al., Intl. J. Cancer, 1998, 75: 63, which is incorporated herein by reference.

The present invention further provides a pharmaceutical composition, which comprises the compound of the present invention or pharmaceutical acceptable salts, hydrates, polycrystalline structures, optical isomers, racemates, diastereomers or enantiomers thereof, and one or more conventional pharmaceutical carriers and/or diluents.

The pharmaceutical composition of the present invention further comprises one or more active drugs in addition to the compound of the present invention or pharmaceutical acceptable salts, hydrates, polycrystalline structures, optical isomers, racemates, diastereomers or enantiomers thereof.

The present invention further provides the use of the compound of the present invention in the preparation of drugs in treating proliferative diseases. The compound of the present invention can be used in the preparation of drugs for inhibiting excessive growth of cells and suspending cell growth. Said proliferative diseases are preferably selected from tumours, multiple sclerosis, rheumatoid arthritis, atherosclerosis and restenosis.

The present invention provdes a method for treating proliferative diseases using the compound of the present invention, said proliferative diseases are preferably selected from tumours, multiple sclerosis, rheumatoid arthritis, atherosclerosis and restenosis.

The present invention furher provides a pharmaceutical composition for treating proliferative diseases, said proliferative diseases are preferably selected from group constituting of tumours, multiple sclerosis, rheumatoid arthritis, atherosclerosis and restenosis.

The compound of the present invention can be of any forms or pharmaceutical acceptable carriers such as ethanol, ethylene glycol (propylene glycol), polyoxyethylene glycol (PEG), Tween, or Solutol etc, pharmaceutical composition may comprise at least one cyclodextrin and acceptable carrier. For example, prodrug and salts and esters of the compound of the present invention. S aid compound may be in any states, such as solid, senii-solid or liquid. The comopound of the present invention can be formulated with pharmaceutical acceptable carriers or diluents into a preparation for oral administration, intravenous administration or subcutaneous administration. Said pharmaceutical composition can be formulated according to standard methods employing solid or liquid carriers, diluents and additives suitable for the desired routes of administration. For oral administration, the compound of the present invention may be administered in the form of troche, capsule, granule, powder. The dosage range of the compound of the present invention is from about 0.05 to 200mg/kg/day, which can be administered in a single dose or in a multiple dose of 2 to 5 portions.

The compound of the present invention can be prepared by other known method for preparing formulation containing drug of low solubility. For example, compounds can be formulated into emulsion with vitamin E and/or PEG polyacrylate derivatives (see WO00/71163 and US6458373 B1). Generally, the compound of the present invention is firstly dissolved in ethanol, followed by addition of vitamin E and/or PEG polyacrylate derivatives to form a therapeutic solution. Ethanol is removed, and a precursor emulsion is formed; or the precursor emulsion can be prepared by adding an aqueous solution comprising surfactants (stablizers). For intravenous injection, the precursor emulsion can be dispersed to form a homogenous emulsion. For oral adminstration, the precursor emulsion is often placed in a gel capsule.

Based on the effects as an inhibitor of tubulin disassembly, the active mechanism of the compound of formula I and that of anti-tumor agents such as paclitaxel and Epothilone is much the same, in which the function of cellular microtubule is disturbed mainly due to the induction of tubulin assembly and stabilization of microtubule assembly, which results in the inhibition of cell division, cell migration and the intercellular signal transmission because all those actions depend on the rapid and effective disassembly of the microtubule. The refore, the compound of formula I is effective for many proliferative diseases, such as solid tumors, liquid tumors, such as leukemia, etc.

The tumors treated by the compounds of the present invention includes head and neck tumors; tumors in liver and gallbladder; breast cancer, ovarian cancer, urogenital cancers, colorectal cancer, lung cancer, brain cancer, kidney cancer, leukemia, gastric carcinoma, liver cancer, glioma, malignant cancers and lymphoma. The method for treating said tumours or cancers comprises administratering a therapeutically effective amount of the compound of the present invention to a cancer patient. The method can be repeated for preventing tumor migration or curing the tumors, if necessary. Especially due to their anti-angiogensis activity, compound of formula I can be used in possible combination of therapeutic agents, especially one or more anti-proliferative, cell growth-inhibiting or cytotoxicity-suppressing compounds. In another aspect, the compound and composition of the present invention can be used in combination with other anti-tumor agents or therapies. In a further aspect, the compounds of the present invention can be used in the treatment of non-carcinoma diseases characterized as cellular hyper-proliferation. In yet another aspect, the compound of the present invention can be used in eluting stent like line-net tubes for suspending cell growth and preventing restenosis or arterial re-blocking. In clinical trial, the compounds of the present invention may lead to one or more of the following phenomena: (i) increase of arterial flow; (ii) alleviation of clinical symptoms of the diseases; (iii) decrease of the rate of restenosis after heart valve surgery; or (iv) prevention/delay the progress of chronic atherosclerosis.

### Examples

### Example 1: Bioconversion for the formation of 14-hydroxylated Epothilone derivatives

A small freezing tube (1ml) of *Streptomyce sp.* strain ATCC55098 is inoculated in 5ml seed medium (20g/L glucose, 20g/L peptone, 10g/L Yeast Extract, pH = 7.0 adjusted with NaOH; used after sterilization), and then cultured in a shaking incubator at 30°C for 2 days. Then, 2.5ml culture is transferred to the fermentation medium (30g/L baker's yeast; 15g/L com syrup, 1g/L CaCO₃, 45g/L cornstarch, 4, 23.8g/L HEPES, 20g/L dextrin, pH = 7.0 adjusted with NaOH; used after sterilization), and cultured at 30°C for 24 hours. 5-10mg Epothilone D or appropriate compounds of the present invention is added to the culture medium, and cultivation is procceded for 2-3 days. The conversion product of 14-hydroxylated Epothilone derivatives are isolated and recovered from the cultures. For example, 14-hydroxy Epothilone D is isolated as the main bioconversion product from Epothilone D of the starting compounds.

The MS (ESI+) of 14-hydroxy Epothilone D (C₂₇H₄₁NO₆S): 508 [M+H]⁺.

### Example 2: Preparation of 15-membered thalidone lactam compound HE1

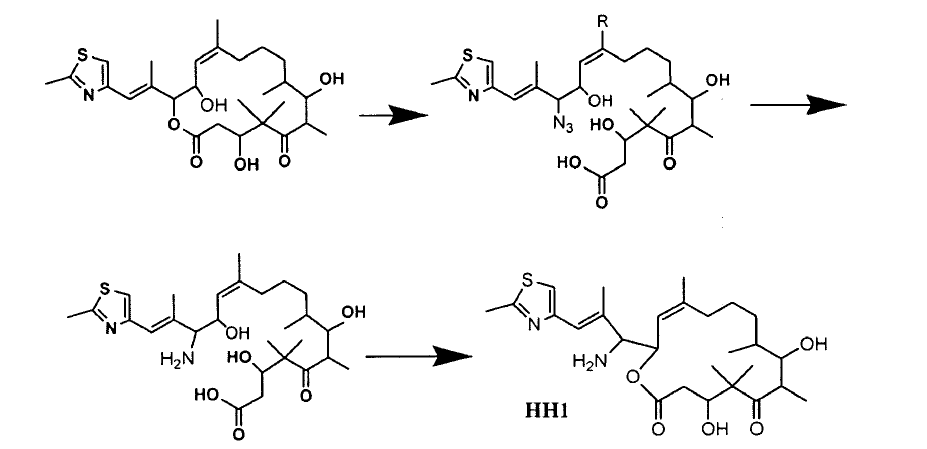

Step 1: A solution of 14-OH Epothilone D (2.62g) in 55ml degassed tetrahydrofuran (THF)/water (10: 1v/v) is treated with a catalytic amount of tetrakis(triphenylphosphine)palladium (0.58g) under argon atomsphere, the suspsension is stirred at 25°C in argon for 30 minutes, and the resulting homogenous solution in light yellow colour is then immediately treated with 25ml of a degassed aqueous solution of sodium azide (0.49g). Reaction is maintained at 45°C for 1 hour, followed by diluting with 50ml water, and extracting with ethyl acetate. The collected extacts are washed with saturated NaCl soltuion, dried over Na₂SO₄, followed by filtration and evaporation, and product is obtained after purified by SiO₂ chromatography.

Step 2a: a solution of the product (565mg, 15-azide) obtained from step 1 above dissolved in 15ml THF/water (10: 1) is treated with a solution of trimethylphosphine (1.0M) in 3ml toluene at ambient temperature under argon atmosphere for 2 hours. The mixture is concentrated, and product is obtained after purified by SiO₂ chromatography.

Step 2b: a solution of the product (565mg, 15-azide) obtained from step 1 above dissolved in 15ml THF/water (10: 1) is treated with triphenylphosphorus (19mg) under argon atmosphere for 2 hours. The mixture is concentrated, and the product is obtained after purified by SiO₂ chromatography.

Step 3: macrocyclic lactonization. At room temperature, tirethylamine and 2,4,6-trichlorobenzoyl chloride are added to the THF solution of the product obtained in step 2. Afte 20 minutes, the mixture is diluted with toluene, and added dropwisely to a warm solution of 4-(dimethylamino)pyridine in toluene within 4 hours. After addition, the mixture is concentrated, and purified by SiO₂ chromatography.
MS (ESI+) of 15-membered thalidone lactam compound HH1 (C₂₇H₄₂N₂O₅S): 507 [M+H]⁺.

### Example 3. Preparation of 15-membered thalidone lactam compound HH2

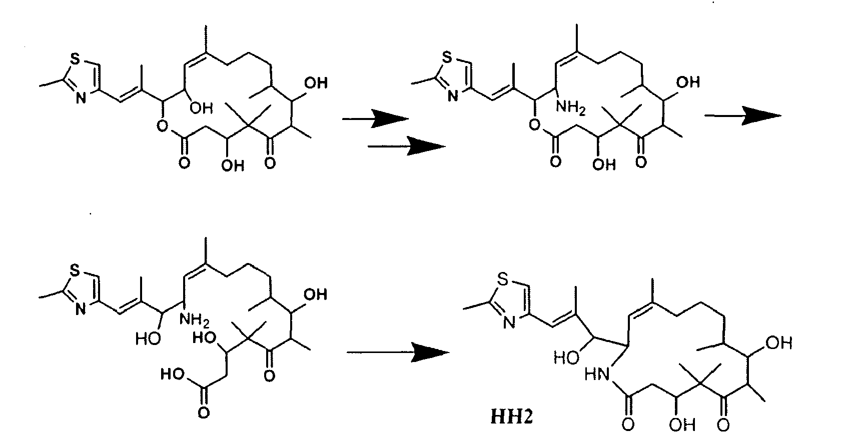

1. A solution of 3,7-O-(tert-butyldimethylsilyl)- 14-OH Epothilone D (30mg) in 1.5ml anhydrous THF is cooled to 0°C. After being treated with diphenylphosphoryl azide (15.5 µl) for 5 minutes, 8.8 µl of 1,8-diazabicyclo(5.4.0)undec-7-ene (DBU) is added, and the reaction is stirred at 0°C for 2 hours, and is then warmed to room temperature and stirred for 20 hours. Subsequently, the solution is added with 30ml ethyl acetate, and washed with water (2 x 10ml), the collected aqueous phase is then extracted with ethyl acetate (2 x 15ml), dried over Na₂SO₄, filtered and evaporated to dry. 3 ,7-O-((tert-butyldimethylsilyl) protected 14-azide Epothilone is then purified by SiO₂ chromatography.
2a. A solution of azide (14mg) (obtained from the above step) in 0.3ml THF and trimethylphosphine (33 µl 1M THF solution) is stirred for 5 minutes, followed by treating with 80 µl of water, and stirring for 3 hours, azide is thoroughly consumed, phosphoryl imine is completely converted to amine upon addition of 50µl of a 28% NH₄OH aqueous solution. After being stirred at 25°C (room temperature) for 1 hour, solvent in the mixture is evaporated under vacuum. Chromatographic separation is performed with silcon gel (10% methanol in chloroform solution) to afford 3,7-O-(tert-butyldimethylsilyl) protected 14-amino Epothilone.
2b. another method: 18mg of Lindlar catalyst is suspended and saturated in 0.5ml ethanol, followed by addition of the azide (obtained from the above step) dissolved in ethanol-methanol mixture, stirred at room temperature for 30minutes, the resulting suspension is filtered through celite, washed with ethyl acetate, dried under vacuum.
3. At room temperature, a methanol solution of the product obtained in step 2 above is treated with 1N NaOH, the reactin is monitored with TLC or HPLC, and reaction is stopped by adding phosphate buffer of pH₄, methanol is removed by vacuum evaporation, aqueous residue is then extacted with ethyl acetate, dried over Na₂SO₄, filtered and evaporated to dry.
4a. the product obtained in step 3 above (540mg) is dissolved in 15ml acetonitrile/dimethylformamide (20: 1v/v) solution, which is then cooled to 0°C, and treated with 1-hydroxybenzotriazole (0.135g) and 1-(3-dimethylaminopropyl)-3-ethylcarbodiimide hydrochloride (0.5g). The resulting mixture is then heated to ambient temperature and kept for 12 hours, followed by diluting with water and extracting with ethyl acetate, the extracts are then sequentially washed with water, saturated NaHCO₃, and saturated NaCl solution, dried over Na₂SO₄, filtered and evaporated to dry. The protected 15-membered thalidone lactam compound is obtained after purified by SiO₂ chromatography. The resulting protected product is then subjected to deprotection by dissolving in 1:1 trifluoroacetic acid and dichloromethane to obtain the end product.
4b. another method: Under OC and Ar atmosphere, a solution of compound obtained in step 2 above (0.33g) in 250ml degassed DMF is treated with solid (0.42g) and diphenylphosphoryl azide (0.54ml), the resulting suspension is stirred at 4°C for 24 hours, followed by diluting with phosphate buffer (250ml, pH7.0) at 0°C, and extracting with ethyl acetate (4 x 100ml), the organic phase is washed with 10% aqueous solution of lithium chloride (2 x 100ml), and then dried over Na₂SO₄, filtered and evaporated to dry. The product is obtained after purfied by SiO₂ chromatography.
MS (ESI+) of 15-membered thalidone lactam compound HH2 (C₂₇H₄₂N₂O₅S): 507 [M+H]⁺.

### Example 4. Preparation of 15-membered thalidone compound HH3

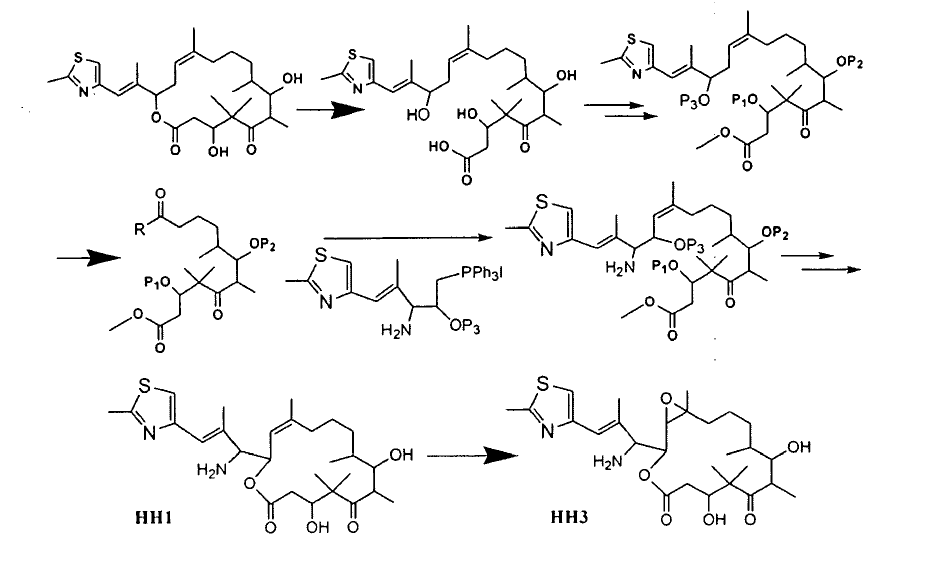

1. Ring opening: Epothilone D (8.4mg) is dissolved in 125µl of DMSO, and diluted with 5ml phosphate buffer (pH7.0), followed by addition of 200 units of Pig liver esterase for overnight hydrolysis at 37°C. pH of the mixture is adjusted to pH=4.5 with 1N HCI, and then extracted with dichloromethane (2 x 5ml), the extracts are dried over Na₂SO₄, filtered and evaporated to dry. The product is obtained by SiO₂ chromatography (eluted with ethyl acetate containing 1% acetic acid). MS (ESI+): 510 [M+H]⁺.
2. Carboxymethylation: product (1 mg) from step 1 above is dissolved in 0.5ml mixture of 2:7 methanol: toluene, followed by addition of 2 drops of trimethylsilyl diazomethane, after 10 minutes at 25°C, mixture is evaporated to dry, and purified by SiO₂ chromatography to obtain the pure product. MS (ESI+): 524 [M+H]⁺.
3. Protection for free hydroxy group: the product obtained in step 2 above (20.4 mg) is dissolved in 2ml anhydrous dichloromethane, followed by addition of 2,6-dimethylpyridine (2,6-lutidine) (23µl), cooled to -14°C, added dropwisely with t-butyldimethysilyl triflate (32µl), Reaction is proceeded for 30 minutes, fol lowed by addition of 2,6-dimethylpyridine (2,6-lutidine) (33µl) and t-butyldimethysilyl triflate (65µl), after reacting for 12 hours, saturated NaHCO₃ (5ml) is added, which is then extracted with dichloromethane (2 x 5ml), the resulting extacts are then dried over Na₂SO₄, filtered and evaporated to dry. The product (tri -3,7,15-TBS-Epo D) is then purified by SiO₂ chromatography. MS(ESI+) : 852 [M+H-CH₃]⁺.
4. Cleavage of molecule: product (6.4mg) from step 3 above is dissolved in 2ml anhydrous dichloromethane, and then cooled to -78°C, ozone is bubbled through the solution for about 2 minutes, the solution become light blue in colour. The solution is then added with triphenylphosphine (8mg) and warmed to room temperature within 30 minutes, the mixture is evaporated to dry, and purified by SiO2 chromatography to afford the pure product (L12 in the case R, R1, R2 is methyl). MS (ESI+): 573 [M+H]⁺.
5. Preparation of phosphonium: small molecule compound L24 (wherein R9 is thiazole, X'=OP3, R15=H) can be prepared according to the general procedure of synthetic route of reaction 9. 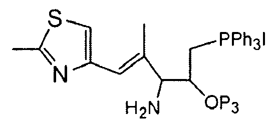
6. Wittig: product (18mg) from step 5 above is dissolved in 0.5ml of anhydrous tetrahydrofuran, and cooled to 0°C, followed by addition of sodium bis(trimethylsilyl)amide (31µl), the solution become brown in colour, the mixture is cooled to -20C, and added with 7.3mg of product obtained in step 4 above dissolved in 0.5ml of anhydrous THF. Aft er reacting for 10 minutes, saturated NaHCO₃ (4ml) is added, which is then extracted with dichloromethane (2 x 2ml), the extracts are then dried over Na₂SO₄, filtered and evaporated to dry. The product is purified by SiO₂ chromatography. MS (ESI+): 867 [M+H-CH₃]⁺.
7. Deptrotection of methyl ester: product (2.2mg) from step 6 above is dissolved in 0.5ml t-butyl alcohol/water (2:1), and treated with 1 M LiOH (40µl), reaction is stirred at room temperature for 48 hours. The product is purified by SiO₂ chromatography.
8. Deprotection of 14-OH (removal of P3): product from step 7 above is dissolved in a mixture of acetonitrile, water and acetic acid. R eation is monitored for the disappearance of the starting material by TLC or HPLC, and is dried by vacuum evaporation. Or product from step 7 is subjected to hydrolysis using desilylating agent or acid in inert solvent or mixture thereof such as TASF or HF in THF. Pyridine is hydrolyzed and selected desilylation is achieved.
9. Macrocyclic lactonization : 87µl of triethylamine and 68µl of 2,4,6-trichlorobenzoyl chloride (Aldrich) are added to a solution of 0.216 g of hydroxy acid product from step 8 in 3ml THF at room temperature. The resulting solution is stirred at 0°C for 1 hour, which is then added dropwisely in 4 hours to a warm solution of 0.354g N,N-(dimethylamino)-pyridine in toluene at room temperature. After addition, the solution is stirred for 2 hours, and concentrated by evaporation. The product is obtained after purified by SiO₂ chromatography.
10. Deprotection: the protected product (67mg) is dissolved in 1.5ml THF, and treated with hydrogen fluoride-pyridine (0.6ml) at 0°C. After 20 minutes, reaction is warmed to room temperature, and kept at this temperature for 5 hours, and then cooled to 0°C again. Methoxytrimethylsilane (6ml) is slowly added, the resulting mixture is warmed to room teimperature, and then subjected to evaporation to afford an oily product, which is then purified by SiO₂ chromatography and a pure product is thus obtained. MS (ESI+) of 15-membered thalidone lactam compound (C₂₇H₄₂N₂O₅S) : 507 [M+H]⁺.
11. Epoxidation : product from step 10 above is subjected to epoxidation according to Example 9 to obtain the 15-membered epoxy thalidone compound HH3 (C₂₇H₄₂N₂O₆S). MS(ESI+) : 523 [M+H]⁺.

### Example 5: Preparatoin of 15-membered thalidone lactam compound HH4

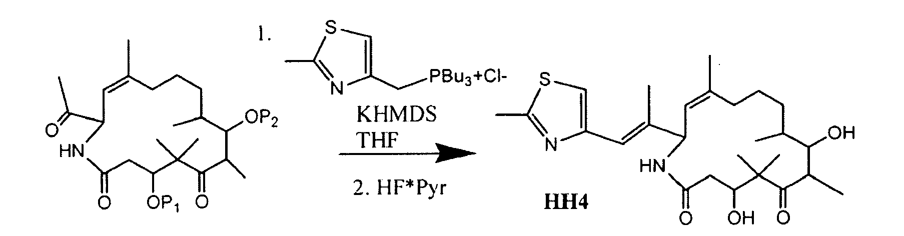

At -30°C, to a solution of 2-methylthizole-4-methyl tri-n-butyl chloride (0.64g) in 3ml THF added dropwisely a solution of 0.5M potassium bis(trimethylsilyl)amide (KHMDS) in toluene (1.5ml) in 10 minutes. The resulting solution is warmed to 0°C in 40 minutes, and cooled to -70°C, followed by dropwise addition of a solution of formula L20 (where Rm is methyl, X is NH in L20) ketone compound (85mg) in 1ml THF (A.Rivkin et.al., J.Am.Chem.Soc. 2003, 125:2899). After 20 minutes, the resulting mixture is warmed to -30°C in 1 hour, and kept at this temperature for 2 hours. Reaction is stopped upon addition of saturated aqueous solution of ammonium chloride, which is then extracted with ethyl acetate, the extracts are then washed with brine water, dried over MgSO₄, filtered and evaporated to dry. The protected product is obtained after purified by SiO₂ chromatography.

The thus protected product (67mg) is dissolved in 1.5ml THF, and treated with hydrogen fluoride-pyridine (0.6ml) at 0°C. After 20 minutes, reaction is warmed to room temperature, and kept at this temperature for 3.5 hours, and then cooled to 0°C again. Methoxytrimethylsilane (6ml)is slowly added, the resulting mixture is warmed to room temperature, and then subjected to evaporation to afford an oily product, which is then purified by SiO₂ chromatography and a pure product is thus obtained. MS (ESI+) of 15-membered thalidone lactam compound HH4 (C₂₆H₄₀N₂O₄S) : 477 [M+H]⁺.

In the case L20 ketone compound, where Rm is methyl, X is O, is used in the reaction, 15-membered thalidone lactam compound HH5 (C₂₆H₃₉NO₅S) is obtained. MS (ESI+): 478 [M+H]⁺.

15-membered thalidone lactam compound HH5 is subjected to epoxidation as shown in equation 15 of the present invention as described in Example 9 to obtain 14-epoxy thalidone compound HH6 (C₂₆H₃₉NO₆S). MS (ESI+): 494 [M+H]⁺.

### Example 6: Preparation of (2-methyl4-thiazole)methyl tributylphosphonium chloride

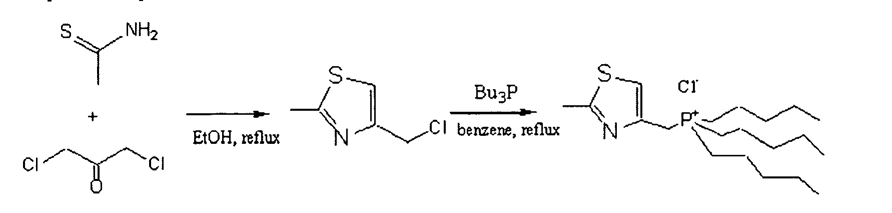

A mixture of 1.3-dichloroacetone (30mmol) and thioacetamide (30mmol) is dissolved in 21ml anhydrous ethanol, and reflux overnight under nitrogen. Solution is concentrated under vacuum, residue is dissolved in water (100ml), pH is adjusted to pH 8.0, followed by extraction with diethyl ether. Sequentially added with saturated NaHCO₃ (4ml), extracted with dichloromethane (2 x 2ml), the collected extacts are washed with saturated NaHCO₃, water, and brine water, the organic phase is dried over Na₂SO₄, filtered and evaporated to dry. The product, 2-methyl-4-chloromethylthiazole, is obtained after purified by SiO₂ chromatography.

To a solution of 2-methyl-4-chloromethylthiazole (557mg) in 3ml benzene dropwisely added tri-n-butylphosphine. The resulting solution is reflux overnight under nitrogen, solution is concentrated under vacuum, and residue is subjected to crystallization by adding a mixture of 1: 1 (v/v) diethyl ether and hexane. S olid is filtered and washed with a small amount of hexane and then dry, to afford phosphonium salt as white solid.

### Example 7: Preparation of 15-membered pyridyl lactam compound HH9

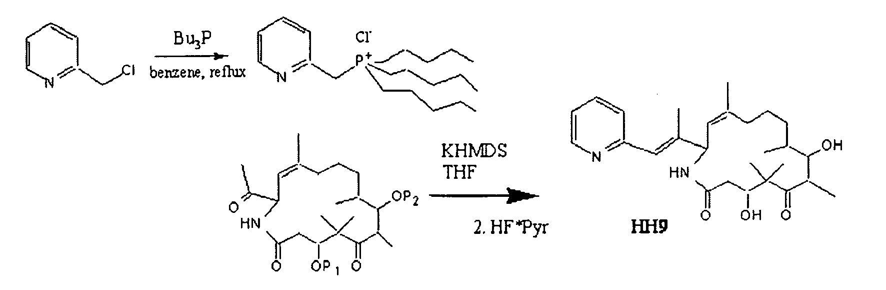

Preparation of (2-pyridyl)methyl tri-n-butylphosphonium chloride. To a solution of 2-(chloromethyl)pyridine (10mmol) in 15ml benzene is added dropwisely tri-n-butylphosphine (10mmol) under nitrogen, the resulting solution is reflux for 18 hours, and then cooled to room temeprature. The solution is concentrated under vacuum. Air is excluded by any means. To the residue is added diethyl ether and a white solid is obtained. F itrate is filtered out. The thus obtained white solid is dried with diethyl ether under vacuum in nitrogen atmosphere, and product is obtained in the form of a white powder.

Compound is prepared according to the procedure of Example 5, (2-pyridyl)methyl tri-n-butyiphosphonium chloride is reacted with formula L20 ketone (in the case X is NH). The solution is warmed to -10°C before the end of the reaction. A pure product, 15-membered pyridyl lactam compound HH9 (C₂₇H₄₀N₂O₄) is obtained. MS (ESI+): 457 [M+H]⁺.

### Example 8: Preparation of benzothiazolephosphonium compound L34 and 14-epoxybenzo thalidone compound HH8

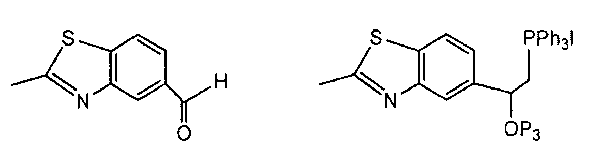

To a solution of dimethylbenzothiazole (Fluka, Buchs) (8 g) in 50ml tetrachloromethane is added N-bromosuccinimide (10.5g), the solution is radiated with a tungsten lamp and heated for 4 hours to 80°C. The resulting mixture is cooled to room temperature, and then filtered, solvent is evaporated. The thus obtained oily matter is mixed with 50% acetic acid aqueous solution (100ml) and hexamethylenetetramine (23.4 g), the mixtue is heated for 2 hours to 110°C. Reaction is then stopped by adding water, and extracted with ethyl acetate. The collected extracts are sequentially extacted with saturated NaHCO₃, water, and brine water, dried over Na₂SO₄, filtered and evaporated to dry. The product is obtained after purified by SiO₂ chromatography.

Dimethylbenzothiazolephosphonium L34 can be prepared according to procedure 2, 3, 4, 5 of synthetic route of reaction 8, i.e. reacting dimethylbenzothiazole aldehyde with vinylmagnesium bromide, P3 protection, Sharpless Reduction, iodization and finally reacting dimethylbenzothiazole iodide with triphenylphosphine.

Benzothiazolephosphonium L34 prepared in the present Example is reacted with compound L12 (compound L12, where R, R1, R2 is methyl) according to the preparation procedure of Example 4 (step 6-11) of the present invention, 14-epxoybenzo thalidone compound HH8 (C₂₇H₃₉NO₆S) is obtained. MS (ESI+): 504 [M+H]⁺.

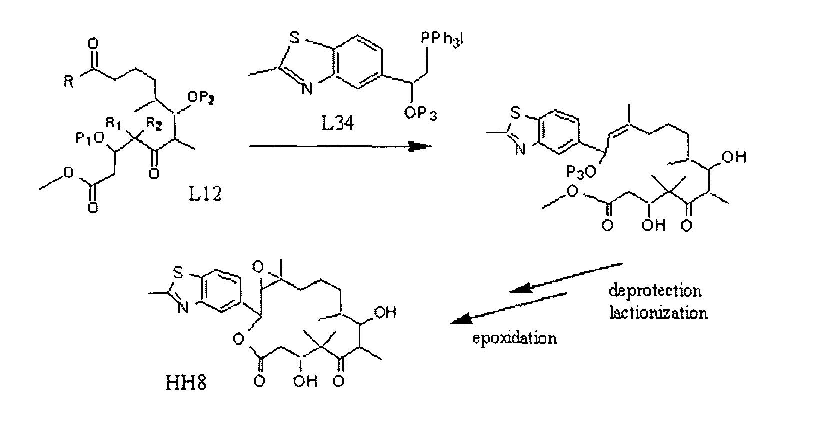

### Example 9: Epoxidation

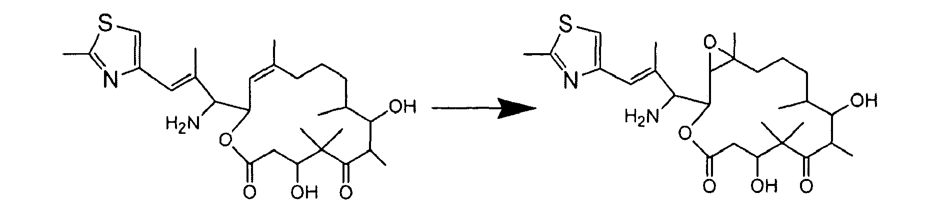

The present example describes the epoxidation reaction of compound of formula I as A-Q links together to form a C=C bond. A t -78°C, to a solution of deoxygenated compound (505mg) of the present invention in 10ml CH₂Cl₂ is added dropwisely a solution of dimethyldiethylene oxide (0.1M in acetion, 17ml). The resulting mixture is heated to -50°C, and kept at this temperatue for 1 hour, followed by addition of another portion of the dimethyldiethylene oxide (5ml), the reaction is proceeded for 1.5 hours at -50°C. Reactants are dired at -50°C under nitrogen vapor. The product is obtained after purified by SiO₂ chromatography.

This general procedure may be suitably applied to other compounds of the present invention for preparing the corresponding 12, 13-epoxy derivatives.

### Example 10:

### Detection of biological activity

In the study, sulforhodamine B (SRB) was used for screening the selected compounds of the present invention according to their anti-tumor activity on four different tumor cell lines. In SRB analysis, the cultured cells were trypsinized, counted and diluted to a suitable concentration (5000-7500 cells/100 µl) with a culture medium. The cells were inoculated into a 96-well microtiter plate at 100 µl suspension/well. The test compounds were diluted in the culture medium to 2 x 1000 nM ∼ 2 x 0.001 nM, and added to each well after 20 hours. The cells were then cultured for 3 days, fixed with 100 µl 10% trichloroacetic acid at 4° C for 1 hour, and then stained with 0.2% SRB/1% acetic acid at room temperature for 20 min. The unbound dye was rinsed with 1% acetic acid. The bound dye was dissolved with 200 µl 10mM Tris-base. The amount of the bound dye was calculated from OD value detected at wavelength of 515 nm. The amount of the bound dye was in direct proportion to the total amount of cell proteins. The data was analyzed with Kaleida Graph Program, and the half inhibitory concentration (IC50) was calculated. Epothilones D and B were detected in parallel for comparison. The results of the cytotoxic experiments on the selectively tested compounds according to the present invention are shown as follows. The other compounds according to the present invention may also be detected with the similar method.

The active mechanism was detected by cell-based tubulin assembly assay. In the assay, MCF-7 cells cultured in a 35 mm dish were treated with 1 µM of the compound according to the present invention at 37° C for 1 hour. The e cells were washed twice with 2 mL PBS containing Ca and Mg, and then were treated with 300 µL lysis solution (20 mM Tris, pH 6.8, 1mM MgCl₂, 2mM EDTA, 1% Triton X-100, with protainase inhibitor) for 5-10min to lyse the cells. The lysate was removed to a 1.5-mL Eppendorf tube, and centrifuged at 18000g for 12 min. The supernatant containing soluble or unassembled tubulin was separated with the granular precipitate containing insoluble or assembled tubulin and removed to a new tube. The granular precipitate was re-suspended with 300 µL lysis solution. Each sample was analyzed by SDS-PAGE and immunoblotting using anti-β-tubulin antigen (Sigma). The amount of β-tubulin on the blotting was analyzed with NIHImage program for detecting the changes of tubulin assembly in the cells.

The tubulin assembly assay demonstrated that 15-membered thiazole derivatives had the same active mechanism as Epothilones, and exhibited similar dynamics and effects under the study condition. The other compounds according to the present invention may also be detected with the same method.

| cCompounds | Half Inhibitory Concentration of Bacterial Growth (IC₅₀, nM) | | | |
|---|---|---|---|---|
| | MCF-7 (Breast Cancer) | NC1/ADR-RES (MDR Breast Cancer) | SF-268 (Glioma) | NCI-H460 (Lung Cancer) |
| Epo D | 13 | 42 | 18 | 17 |
| Epo B | 0.5 | 5 | 0.8 | 0.7 |
| 15-aminoexpoxythiazone HH3 | 1 | 3 | 1 | 0.5 |
| 14-expoxythiazone HH6 | 0.2 | 1 | 1 | 0.5 |
| 14-expoxybenzothiazolinone HH8 | 0.5 | 0.5 | 1 | 0.2 |

## Claims

1. A 15-membered thalidone compound of the following general formula I: 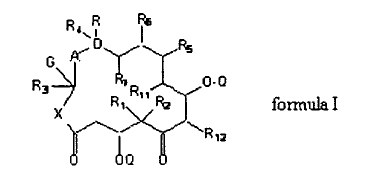 wherein,
as A-D represents a C=C bond of formula (a) or an epoxy group of formula (b), R₄ is not exist, 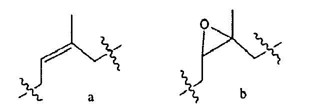
as A-D represents a C-C bond, R₄ represents a hydroxy group or H,
G is selected from a substituted or unsubstituted alkyl group, a substituted or unsubstituted aryl group, a heteroaryl group, a heterocyclic group, a cycloalkyl group, or any one selected from the following formulae: 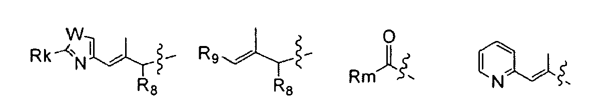 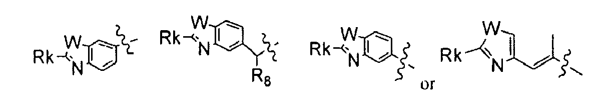
Q is selected from H, a C₁₋₄ alkyl group, NH₂ or a hydroxy-protecting group;
R₁, R₂ are each independently selected from H or a substituted or unsubstituted C₁₋₄ alky group, or together form a cycloalkyl group;
R₈ is selected from H, a hydroxy group, a substituted or unsubstituted C₁₋₆ alkyl group or NH₂, N₃ or NR₁₃R₁₄;
X represents O, S or N-R₁₅, wherein R₁₅ represents H, NR₁₆R₁₇, a substituted or unsubstituted C₁₋₄alkyl group, a substituted or unsubstituted aryl group, a cycloalkyl group or a heterocyclic group;
R₉ is selected from H, a substituted or unsubstituted C₁₋₄ alkyl, an aryl group, a heteroaryl group, a cycloalkyl group or a heterocyclic group;
R₁₂ is selected from H, an allyl group, a hydroxy group, NH₂ or a substituted or unsubstituted C₁₋₆ alkyl;
Rm is selected from H, methyl, NR₁₆R₁₇ or halomethyl;
Rk is selected from H, a substituted or unsubstituted C₁₋₄ alkyl group, an aminoalkyl group, a hydroxyalkyl group or a haloalkyl;
R₃, R₄, R₅, R₆, R₇, R₁₁, R₁₃, R₁₄, R₁₆, R₁₇ are each independently selected from H, a hydroxy group, NH₂ or a substituted or unsubstituted C₁₋₆ alkyl group, wherein R₅, R₆ may together form a C=C bond;
R is selected from H, trifluoromethyl, a substituted or unsubstituted alkyl group or halogen;
W represents S or O, NH, N-alkyl:
or pharmaceutical acceptable salts, hydrates, polycrystalline structures, optical isomers, racemates, diastereomers or enantiomers of said compound.

2. The compound according to claim 1, **characterized in that** G in said compound is selected from: 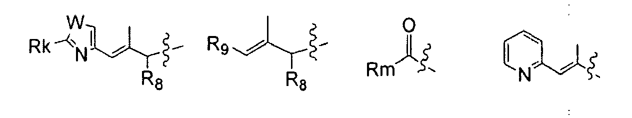 or

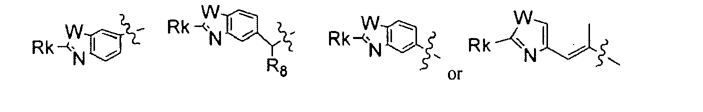

3. The compound according to claim 1, **characterized in that** said compound is represented by the structure of the following general formula II: 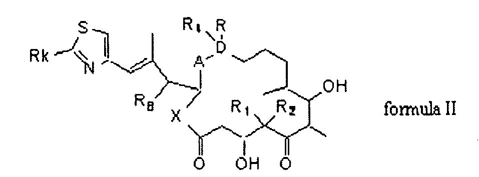
wherein, X is NR₁₅ or O;
R₈ is NHR₁₅ or OQ;
each of the other groups has the same meaning as defined in claim 1.

4. The compound according to claim 1, **characterized in that** said compound is represented by the structure of the following general formula III: 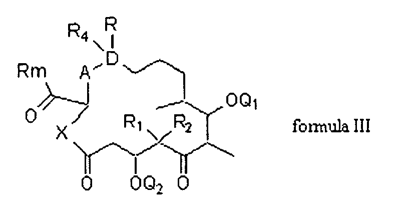 wherein, Q₁ and Q₂ each independently represents H, a C₁₋₄alkyl group, NH₂ or a hydroxy-protecting group; each of the other groups has the same meaning as defined in claim 1.

5. The compound according to claim 1, **characterized in that** said compound is represented by the structure of the following general formula IV: 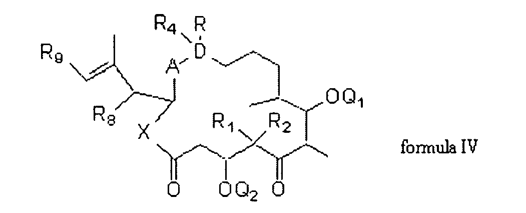 wherein, each group has the same meaning as defined in claim 1.

6. The compound according to claim 1, **characterized in that** said compound is represented by the structure of the following general formula V: 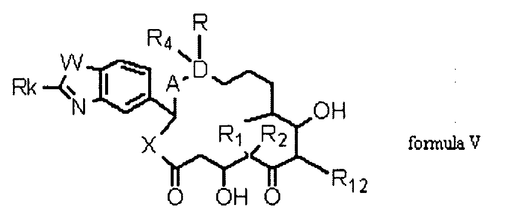 wherein, X is NR₁₅ or O; R₁₅ is H, a methoxy group or an alkyl group; R₁₂ is H, an allyl group, a substituted or unsubstituted C₁₋₆ alkyl group.

7. The compound according to claim 1, **characterized in that** said compound is selected from the following compounds:

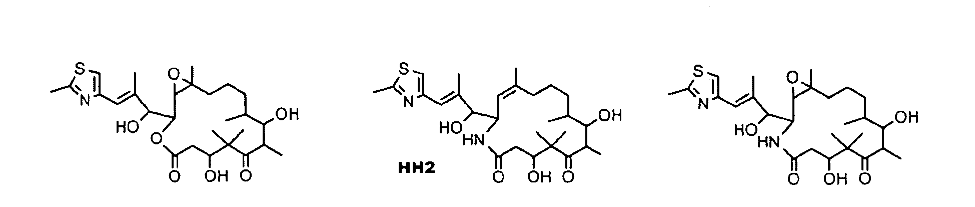

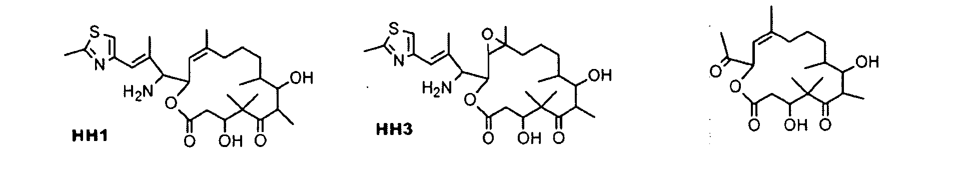

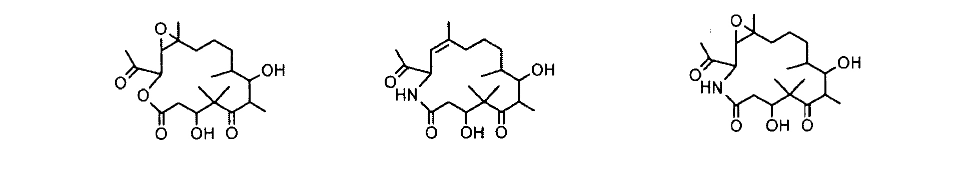

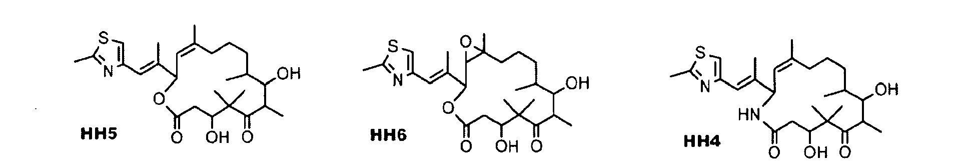

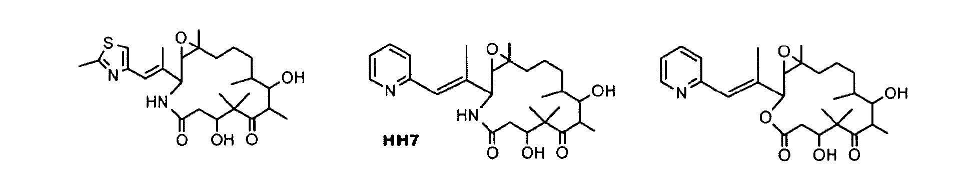

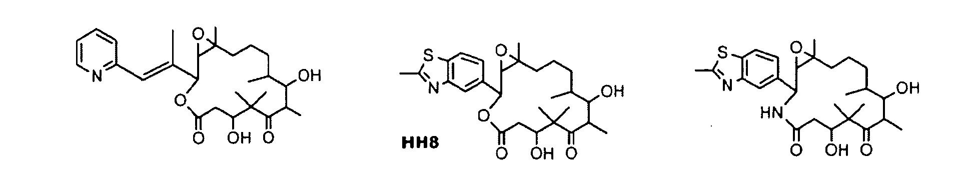

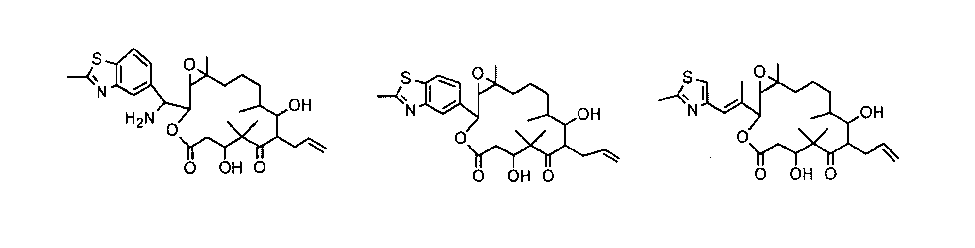

8. The compound of the following general formula VI: 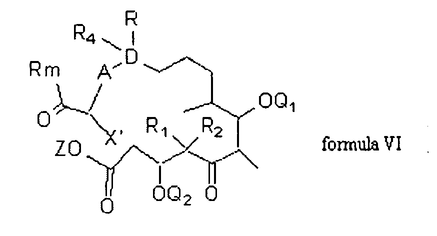 wherein
X' is NHR₁₅, NR₁₅P, OH or OQ; wherein, R₁₅ is H, a methoxy group or an alkyl group; P is a N-protecting group;
Z is H, a substituted or unsubstituted alkyl group, a cycloalkyl group, an aryl group or a carboxyl-protecting group;
Q₁ and Q₂ each independently represents H, a C₁₋₄ alkyl group, NH₂ or a hydroxy-protecting group.

9. A pharmaceutical composition, **characterized in that** said pharmaceutical composition comprises compound of any one of claims 1 to 7, or pharmaceutical acceptable salts, hydrates, polycrystalline structures, optical isomers, racemates, diastereomers or enantiomers thereof, and one or more pharmaceutical carriers and/or diluents.

10. A composition according to claim 9, **characterized in that** said composition further comprises one or more active drugs in addition to compound of any one of claims 1 to 7, or pharmaceutical acceptable salts, hydrates, polycrystalline structures, optical isomers, racemates, diastereomers or enantiomers thereof.

11. Use of compound of any one of claims 1 to 7 in the preparation of drugs for treating proliferative diseases.

12. Use of compound of claim 11, **characterized in that** said proliferative diseases are selected from groups constituting of tumours, multiple sclerosis, rheumatoid arthritis, atherosclerosis and restenosis.

13. A method for preparing compounds of formula I of claim 1:
wherein, 15-membered thalidone lactone compounds or 15-membered thalidone lactam compounds of formula I are prepared according to synthetic route of reaction 2 to 4 using 14-hydroxy Epothilone, in which a 15-membered ring is subjected to macrocyclic lactonization or macrocyclic lactamization;
compound L12 is obtained from Epothilone D and derivatives thereof LL according to synthetic route of reaction 5, said compounds and phosphonium of formula L13 or L17 or compound L34 are subjected to 15-membered macrocyclic lactonization or lactamization to obtain compound L16 or L21 (synthetic route of reaction 5 and 6) or HH8;
compound L32 can also be prpepard by total syntheis as shown in synthetic route of reaction 12 and 12B using compound L31-A, L31-B (L12).
